# EUROPEAN PATENT APPLICATION

(11) **EP 1 391 462 A1**
(43) Date of publication of application: **25.02.2004**
(21) Application number: 02722733.9
(22) Date of filing: 24.04.2002
(51) Int. Cl.: C07K 7/06, C07K 7/08, C07K 14/415, C07K 14/47, G01N 33/53

(54) **CROHN'S DISEASE ANTIBODY-BINDING PEPTIDE AND METHOD OF EXAMINING CROHN'S DISEASE**

(30) Priority: 24.04.2001 JP 2001126121; 25.02.2002 JP 2002047384
(71) Applicant: OTSUKA PHARMACEUTICAL CO., LTD., Chiyoda-ku Tokyo 101-8535 (JP)
(72) Inventor: SAITO, Hiroshi, Itano-gun, Tokushima 771-0203 (JP); KATSURAGI, Kiyonori, Itano-gun, Tokushima 771-1507 (JP); TACHIKAWA, Tetsuya, Itano-gun, Tokushima 771-0206 (JP); TANAKA, Michinori, Itano-gun, Tokushima 771-0203 (JP); OGINO, Koichi, Naruto-shi, Tokushima 772-0003 (JP); TAKI, Takao, Itano-gun, Tokushima 771-0205 (JP)
(74) Representative: HOFFMANN - EITLE
(86) International application number: PCT/JP2002/004061
(87) International publication number: WO 2002/088175

(57) **Abstract**

The invention provides that examination reagents useful in diagnosing Crohn's disease and a method of conveniently and accurately detecting the occurrence of Crohn's disease. The examination reagents contain as the active ingredient a Crohn's disease antibody-binding protein as defined in the following (a) or (b): (a) a peptide consisting of an amino acid sequence selected from among the amino acid sequences represented by SEQ ID NOS:1 to 4; and (b) a peptide consisting of an amino acid sequence derived from the above-mentioned amino acid sequence (a) by substitution, deletion or addition of one to several amino acids and being capable of binding to the Crohn's disease antibody. The examination method can be carried out by detecting the presence/absence of an antibody recognizing human vacuolar H⁺-transport ATPase, a human nuclear protein (Homo sapiens kruppel-like zinc finger protein 300) or rice allergen in a biological sample of a subject.

SEQUENCE LISTING

<110> OTSUKA PHARMACEUTICAL CO.,LTD.
<120> Crohn's disease antibody-binding peptide and method for detecting
Crohn's disease
<130> P02-27
<150> JP 2001/126121
<151> 2001-04-24
<150> JP 2002/47384
<151> 2002-02-25

<160> 51
<170> PatentIn Ver. 2.1

<210> 1
<211> 9
<212> PRT
<213> phage library
<400> 1
Gly Leu Leu Ala Gln Gln Met Asp Tyr
 1               5

<210> 2
<211> 9
<212> PRT
<213> phage library
<400> 2
Tyr Arg Trp Leu Pro Pro Ser Ser Ala
 1               5

<210> 3
<211> 9
<212> PRT

<213> phage library
<400> 3
Arg Gln Ser Asp Gly Gln Tyr Gln Met
1               5

<210> 4
<211> 9
<212> PRT
<213> phage library
<400> 4
Gly Gly Ile Tyr Gln Asp Leu Val Ser
1               5

<210> 5
<211> 17
<212> PRT
<213> phage library
<400> 5
Ala Glu Gly Glu Leu Gly Leu Leu Ala Gln Gln Met Asp Tyr Ala
1               5                   10                  15
Asp Pro
    17

<210> 6
<211> 18
<212> PRT
<213> phage library
<400>6
Ala Glu Gly Glu Leu Gly Leu Leu Ala Gln Gln Met Asp Tyr Ala
1               5                   10                  15
Asp Pro Ala
        18

<210> 7
<211> 9
<212> PRT
<213> phage library
<400> 7
Arg Leu Val Gly Gln Gln Val Met Gln
 1               5

<210> 8
<211> 17
<212> PRT
<213> phage library
<400> 8
Ala Glu Gly Glu Leu Arg Leu Val Gly Gln Gln Val Met Gln Gly
 1               5                   10                  15
Asp Pro
    17

<210>9
<211> 18
<212> PRT
<213> phage library
<400> 9
Ala Glu Gly Glu Leu Arg Leu Val Gly Gln Gln Val Met Gln Gly
 1               5                   10                  15
Asp Pro Ala
        18

<210> 10
<211> 7
<212> PRT
<213> Homo sapiens
<220> Vacuolar ATPase subunit E

<222> 202...208

<400> 10

Leu Ile Ala Gln Gln Met Met
 1               5

<210> 11

<211> 14

<212> PRT

<213> Homo sapiens

<220> Vacuolar ATPase subunit E

<222> 199...212

<400> 11

Arg Leu Asp Leu Ile Ala Gln Gln Met Met Pro Glu Val Arg
 1               5                   10              14

<210> 12

<211> 9

<212> PRT

<213> phage library

<400> 12

Arg Ala Gln Gln Val Val Glu Phe Ser
 1               5

<210> 13

<211> 18

<212> PRT

<213> phage library

<400> 13

Ala Glu Gly Glu Leu Arg Ala Gln Gln Val Val Glu Phe Ser Gly
 1               5                   10                  15

Asp Pro Ala
        18

<210> 14
<211> 226
<212> PRT
<213> Homo sapiens
<220> Vacuolar ATPase subunit E
<400> 14

<210> 15
<211> 18
<212> PRT
<213> phage library
<400> 15
Ala Glu Gly Glu Leu Tyr Arg Trp Leu Pro Pro Ser Ser Ala Gly
1               5                   10                  15
Asp Pro Ala
18

<210> 16
<211> 9
<212> PRT
<213> phage library
<400> 16
Asp Arg Trp Leu Pro Glu Gly Asp Gly
1               5

<210> 17
<211> 18
<212> PRT
<213> phage library
<400> 17
Ala Glu Gly Glu Leu Asp Arg Trp Leu Pro Glu Gly Asp Gly Gly
1               5                   10                  15
Asp Pro Ala
18

<210> 18

<211> 9
<212> PRT
<213> phage library
<400> 18
His Glu Trp Leu Pro Leu Tyr Asp Ala
1               5

<210> 19
<211> 18
<212> PRT
<213> phage library
<400> 19
Ala Glu Gly Glu Leu His Glu Trp Leu Pro Leu Tyr Asp Ala Gly
1               5                   10                  15
Asp Pro Ala
        18

<210> 20
<211> 18
<212> PRT
<213> phage library
<400> 20
Ala Glu Gly Glu Leu Arg Gln Ser Asp Gly Gln Tyr Gln Met Gly
1               5                   10                  15
Asp Pro Ala
        18

<210> 21
<211> 14
<212> PRT
<213> phage library
<400> 21
Ala Glu Gly Glu Leu Arg Gln Ser Asp Gly Gln Tyr Gln Met

5                   10

<210> 22
<211> 14
<212> PRT
<213> phage library
<400> 22
Ser Glu Gly Glu Leu Arg Gln Ser Asp Gly Gln Tyr Gln Met
                5                   10

<210> 23
<211> 14
<212> PRT
<213> phage library
<400> 23
Ala Ala Gly Glu Leu Arg Gln Ser Asp Gly Gln Tyr Gln Met
                5                   10

<210> 24
<211> 14
<212> PRT
<213> phage library
<400> 24
Ala Glu Ala Glu Leu Arg Gln Ser Asp Gly Gln Tyr Gln Met
                5                   10

<210> 25
<211> 14
<212> PRT
<213> phage library
<400> 25
Ala Glu Gly Ala Leu Arg Gln Ser Asp Gly Gln Tyr Gln Met
                5                   10

<210> 26
<211> 14
<212> PRT
<213> phage library
<400> 26
Ala Glu Gly Glu Ala Arg Gln Ser Asp Gly Gln Tyr Gln Met
                5                   10

<210> 27
<211> 14
<212> PRT
<213> phage library
<400> 27
Ala Glu Gly Glu Leu Ala Gln Ser Asp Gly Gln Tyr Gln Met
                5                   10

<210> 28
<211> 14
<212> PRT
<213> phage library
<400> 28
Ala Glu Gly Glu Leu Arg Gln Ala Asp Gly Gln Tyr Gln Met
                5                   10

<210> 29
<211> 14
<212> PRT
<213> phage library
<400> 29
Ala Glu Gly Glu Leu Arg Gln Ser Asp Gly Gln Ala Gln Met
                5                   10
<210> 30

<211> 14
<212> PRT
<213> phage library
<400> 30
Ala Glu Gly Glu Leu Arg Gln Ser Asp Gly Gln Tyr Gln Ala
                5                   10

<210> 31
<211> 13
<212> PRT
<213> Homo sapiens
<220> kruppel-like zinc finger protein 300
<222> 126...138
<400> 31
Lys Val Cys Gln Gly Asp Gly Gln Leu Gln Arg Phe Leu
                5                   10

<210> 32
<211> 604
<212> PRT
<213> Homo sapiens
<220> kruppel-like zinc finger protein 300
<400> 32
<400> 1
Met Met Lys Ser Gln Gly Leu Val Ser Phe Lys Asp Val Ala Val Asp
  1               5                   10                  15
Phe Thr Gln Glu Glu Trp Gln Gln Leu Asp Pro Ser Gln Arg Thr Leu
             20                  25                  30
Tyr Arg Asp Val Met Leu Glu Asn Tyr Ser His Leu Val Ser Met Gly
         35                  40                  45
Tyr Pro Val Ser Lys Pro Asp Val Ile Ser Lys Leu Glu Gln Gly Glu
     50                  55                  60
Glu Pro Trp Ile Ile Lys Gly Asp Ile Ser Asn Trp Ile Tyr Pro Asp

65                  70                  75                  80
Glu Tyr Gln Ala Asp Gly Arg Gln Asp Arg Lys Ser Asn Leu His Asn
                85                  90                  95
Ser Gln Ser Cys Ile Leu Gly Thr Val Ser Phe His His Lys Ile Leu
            100                 105                 110
Lys Gly Val Thr Arg Asp Gly Ser Leu Cys Ser Ile Leu Lys Val Cys
        115                 120                 125
Gln Gly Asp Gly Gln Leu Gln Arg Phe Leu Glu Asn Gln Asp Lys Leu
    130                 135                 140
Phe Arg Gln Val Thr Phe Val Asn Ser Lys Thr Val Thr Glu Ala Ser
145                 150                 155                 160
Gly His Lys Tyr Asn Pro Leu Gly Lys Ile Phe Gln Glu Cys Ile Glu
                165                 170                 175
Thr Asp Ile Ser Ile Gln Arg Phe His Lys Tyr Asp Ala Phe Lys Lys
            180                 185                 190
Asn Leu Lys Pro Asn Ile Asp Leu Pro Ser Cys Tyr Lys Ser Asn Ser
        195                 200                 205
Arg Lys Lys Pro Asp Gln Ser Phe Gly Gly Gly Lys Ser Ser Ser Gln
    210                 215                 220
Ser Glu Pro Asn Ser Asn Leu Glu Lys Ile His Asn Gly Val Ile Pro
225                 230                 235                 240
Phe Asp Asp Asn Gln Cys Gly Asn Val Phe Arg Asn Thr Gln Ser Leu
                245                 250                 255
Ile Gln Tyr Gln Asn Val Glu Thr Lys Glu Lys Ser Cys Val Cys Val
            260                 265                 270
Thr Cys Gly Lys Ala Phe Ala Lys Lys Ser Gln Leu Ile Val His Gln
        275                 280                 285
Arg Ile His Thr Gly Lys Lys Pro Tyr Asp Cys Gly Ala Cys Gly Lys
    290                 295                 300
Ala Phe Ser Glu Lys Phe His Leu Val Val His Gln Arg Thr His Thr
305                 310                 315                 320
Gly Glu Lys Pro Tyr Asp Cys Ser Glu Cys Gly Lys Ala Phe Ser Gln
                325                 330                 335

Lys Ser Ser Leu Ile Ile His Gln Arg Val His Thr Gly Glu Lys Pro
            340                 345                 350
Tyr Glu Cys Ser Glu Cys Gly Lys Ala Phe Ser Gln Lys Ser Pro Leu
        355                 360                 365
Ile Ile His Gln Arg Ile His Thr Gly Glu Lys Pro Tyr Glu Cys Arg
    370                 375                 380
Glu Cys Gly Lys Ala Phe Ser Gln Lys Ser Gln Leu Ile Ile His His
385                 390                 395                 400
Arg Ala His Thr Gly Glu Lys Pro Tyr Glu Cys Thr Glu Cys Gly Lys
                405                 410                 415
Ala Phe Cys Glu Lys Ser His Leu Ile Ile His Lys Arg Ile His Thr
            420                 425                 430
Gly Glu Lys Pro Tyr Lys Cys Ala Gln Cys Glu Glu Ala Phe Ser Arg
        435                 440                 445
Lys Thr Glu Leu Ile Thr His Gln Leu Val His Thr Gly Glu Lys Pro
    450                 455                 460
Tyr Glu Cys Thr Glu Cys Gly Lys Thr Phe Ser Arg Lys Ser Gln Leu
465                 470                 475                 480
Ile Ile His Gln Arg Thr His Thr Gly Glu Lys Pro Tyr Lys Cys Ser
                485                 490                 495
Glu Cys Gly Lys Ala Phe Cys Gln Lys Ser His Leu Ile Gly His Gln
            500                 505                 510
Arg Ile His Thr Gly Glu Lys Pro Tyr Ile Cys Thr Glu Cys Gly Lys
        515                 520                 525
Ala Phe Ser Gln Lys Ser His Leu Pro Gly His Gln Arg Ile His Thr
    530                 535                 540
Gly Glu Lys Pro Tyr Ile Cys Ala Glu Cys Gly Lys Ala Phe Ser Gln
545                 550                 555                 560
Lys Ser Asp Leu Val Leu His Gln Arg Ile His Thr Gly Glu Arg Pro
                565                 570                 575
Tyr Gln Cys Ala Ile Cys Gly Lys Ala Phe Ile Gln Lys Ser Gln Leu
            580                 585                 590
Thr Val His Gln Arg Ile His Thr Val Val Lys Ser

595                    600

<210> 33
<211> 18
<212> PRT
<213> phage library
<400> 33
Ala Glu Gly Glu Leu Gly Gly Ile Tyr Gln Asp Leu Val Ser Gly
 1               5                   10                  15
Asp Pro Ala
        18

<210> 34
<211> 14
<212> PRT
<213> phage library
<400> 34
Ala Glu Gly Glu Leu Gly Gly Ile Tyr Gln Asp Leu Val Ser
             5                   10

<210> 35
<211> 14
<212> PRT
<213> phage library
<400> 35
Ser Glu Gly Glu Leu Gly Gly Ile Tyr Gln Asp Leu Val Ser
             5                   10

<210> 36
<211> 14
<212> PRT
<213> phage library
<400> 36

Ala Ala Gly Glu Leu Gly Gly Ile Tyr Gln Asp Leu Val Ser
                5                   10

<210> 37
<211> 14
<212> PRT
<213> phage library
<400> 37
Ala Glu Ala Glu Leu Gly Gly Ile Tyr Gln Asp Leu Val Ser
                5                   10

<210> 38
<211> 14
<212> PRT
<213> phage library
<400> 38
Ala Glu Gly ALa Leu Gly Gly Ile Tyr Gln Asp Leu Val Ser
                5                   10

<210> 39
<211> 14
<212> PRT
<213> phage library
<400> 39
Ala Glu Gly Glu Leu Gly Gly Ile Tyr Ala Asp Leu Val Ser
                5                   10
<210> 40
<211> 14
<212> PRT
<213> phage library
<400> 40
Ala Glu Gly Glu Leu Gly Gly Ile Tyr Gln Asp Leu Ala Ser
                5                   10

<210> 41
<211> 14
<212> PRT
<213> phage library
<400> 41
Ala Glu Gly Glu Leu Gly Gly Ile Tyr Gln Asp Leu Val Ala
                5                   10

<210> 42
<211> 13
<212> PRT
<213> Rice seed
<220> Rice seed allergen RA14
<222> 99...111
<400> 42
His Met Val Gly Gly Ile Tyr Arg Glu Leu Gly Ala Thr
                5                   10

<210> 43
<211> 157
<212> PRT
<213> Rice
<220> Rice allergen
<400> 43
Met Ala Ser Asn Lys Val Val Phe Ser Val Leu Leu Leu Ala Val Val
1               5                   10                  15
Ser Val Leu Ala Ala Thr Ala Thr Met Ala Glu Tyr His His Gln Asp
            20                  25                  30
Gln Val Val Tyr Thr Pro Gly Pro Leu Cys Gln Pro Gly Met Gly Tyr
        35                  40                  45
Pro Met Tyr Pro Leu Arg Val Ala Gly Val Gly Glu Ala Pro Leu Leu
    50                  55                  60
Gly Arg Ala Arg Pro Arg Arg Arg Ala Val Pro Gly Asp Cys Cys Arg

65                  70                  75                  80
Gln Phe Pro Pro Val Asp Tyr Ser Trp Cys Arg Cys Glu Ala Ile Ser
                85                  90                  95
His Met Leu Gly Gly Ile Tyr Arg Glu Leu Gly Ala Pro Asp Val Gly
            100                 105                 110
His Pro Met Ser Glu Val Phe Arg Gly Cys Arg Arg Gly Thr Trp Ser
        115                 120                 125
Ala Arg Arg Arg Ala Pro Gly Val Leu Gln Val Asp Ile Pro Asn Gly
    130                 135                 140
Gly Gly Gly Val Cys Tyr Trp Leu Ala Arg Ser Gly Tyr
145                 150                 155

<210> 44
<211> 157
<212> PRT
<213> Rice seed
<220> Rice seed allergen RA5
<400> 44
Met Ala Ser Asn Lys Val Val Phe Ser Val Leu Leu Leu Ala Val Val
 1               5                  10                  15
Ser Val Leu Ala Ala Thr Ala Thr Met Ala Glu Tyr His His Gln Asp
            20                  25                  30
Gln Val Val Tyr Thr Arg Ala Arg Cys Gln Pro Gly Met Gly Tyr Pro
        35                  40                  45
Met Tyr Ser Leu Pro Arg Cys Arg Ala Leu Val Lys Arg Gln Cys Arg
    50                  55                  60
Gly Ser Ala Ala Ala Ala Glu Gln Val Arg Arg Asp Cys Cys Arg Gln
65                  70                  75                  80
Leu Ala Ala Val Asp Asp Ser Trp Cys Arg Cys Glu Ala Ile Ser His
                85                  90                  95
Met Leu Gly Gly Ile Tyr Arg Glu Leu Gly Ala Pro Asp Val Gly His
            100                 105                 110
Pro Met Ser Glu Val Phe Arg Gly Cys Arg Arg Gly Asp Leu Glu Arg

115                 120                 125
Ala Ala Ala Ser Leu Pro Ala Phe Cys Asn Val Asp Ile Pro Asn Gly
    130                 135                 140
Gly Gly Gly Val Cys Tyr Trp Leu Ala Arg Ser Gly Tyr
145                 150                 155

<210> 45
<211> 160
<212> PRT
<213> Rice
<220> Rice allergen allergen RA5B precursor
<400> 45
Met Ala Ser Asn Lys Val Val Phe Ser Val Leu Leu Leu Ala Val Val
 1               5                  10                  15
Ser Val Leu Ala Ala Thr Ala Thr Met Ala Glu Tyr His His Gln Asp
            20                  25                  30
Gln Val Val Tyr Thr Pro Ala Pro Leu Cys Gln Pro Gly Met Gly Tyr
        35                  40                  45
Pro Met Tyr Pro Leu Pro Arg Cys Arg Ala Leu Val Lys Arg Gln Cys
    50                  55                  60
Val Gly Arg Gly Thr Ala Ala Ala Ala Glu Gln Val Arg Arg Asp Cys
65                  70                  75                  80
Cys Arg Gln Leu Ala Ala Val Asp Asp Ser Trp Cys Arg Cys Glu Ala
                85                  90                  95
Ile Ser His Met Leu Gly Gly Ile Tyr Arg Glu Leu Gly Ala Pro Asp
            100                 105                 110
Val Gly His Pro Met Ser Glu Val Phe Arg Gly Cys Arg Arg Gly Asp
        115                 120                 125
Leu Glu Arg Ala Ala Ala Ser Leu Pro Ala Phe Cys Asn Val Asp Ile
    130                 135                 140
Pro Asn Gly Gly Gly Gly Val Cys Tyr Trp Leu Ala Arg Ser Gly Tyr
145                 150                 155                 160

<210> 46
<211> 165
<212> PRT
<213> Rice seed
<220> Rice seed allergen RA14
<400> 46
Met Ala Ser Asn Lys Val Val Phe Ser Ala Leu Leu Leu Ile Ile Val
1               5                   10                  15
Ser Val Leu Ala Ala Thr Thr Arg Met Ala Asp His His Lys Asp Gln
            20                  25                  30
Val Val Tyr Ser Leu Gly Glu Arg Cys Gln Pro Gly Met Gly Tyr Pro
        35                  40                  45
Met Tyr Ser Leu Pro Arg Cys Arg Ala Val Val Lys Arg Gln Cys Val
    50                  55                  60
Gly Thr Arg Ser Pro Gly Ala Val Asp Glu Gln Leu Ala Gln Asp Cys
65                  70                  75                  80
Cys Arg Glu Leu Ala Ala Val Asp Asp Ser Trp Cys Arg Cys Ser Ala
                85                  90                  95
Leu Asn His Met Val Gly Gly Ile Tyr Arg Glu Leu Gly Ala Thr Asp
            100                 105                 110
Val Gly His Pro Met Ala Glu Val Phe Pro Gly Cys Arg Arg Gly Asp
        115                 120                 125
Leu Glu Arg Ala Ala Ala Ser Leu Pro Ala Phe Cys Asn Val Asp Ile
    130                 135                 140
Pro Asn Gly Thr Gly Gly Val Cys Tyr Trp Leu Gly Tyr Pro Arg Thr
145                 150                 155                 160
Pro Arg Thr Gly His
                165
<210> 47
<211> 166
<212> PRT
<213> Rice
<220> Rice allergen RA14B precursor

<400> 47
Met Ala Ser Asn Lys Val Val Phe Ser Ala Leu Leu Leu Ile Ile Val
1               5                   10                  15
Ser Val Leu Ala Ala Thr Gly Pro Met Ala Asp His His Lys Asp Gln
            20                  25                  30
Val Val Tyr Ser Leu Gly Glu Arg Cys Gln Pro Gly Met Gly Tyr Pro
        35                  40                  45
Met Tyr Ser Leu Pro Arg Cys Arg Ala Val Val Lys Arg Gln Cys Val
    50                  55                  60
Ala Thr Ala His Pro Ala Ala Arg Gly Asn Glu Gln Leu Arg Gln Asp
65                  70                  75                  80
Cys Cys Arg Gln Leu Ala Ala Val Asp Asp Ser Trp Cys Arg Cys Ser
                85                  90                  95
Ala Leu Asn His Met Val Gly Gly Ile Tyr Arg Glu Leu Gly Ala Thr
            100                 105                 110
Asp Val Gly His Pro Met Ala Glu Val Phe Pro Gly Cys Arg Arg Gly
        115                 120                 125
Asp Leu Glu Arg Ala Ala Ala Ser Leu Pro Ala Phe Cys Asn Val Asp
    130                 135                 140
Ile Pro Asn Gly Thr Gly Gly Val Cys Tyr Trp Leu Gly Tyr Pro Arg
145                 150                 155                 160
Thr Pro Arg Thr Gly His
                165

<210> 48
<211> 166
<212> PRT
<213> Rice seed
<220> Rice seed allergen RAG2
<400> 48
Met Ala Ser Asn Lys Val Val Phe Ser Ala Leu Leu Leu Ile Ile Val
1               5                   10                  15
Ser Val Leu Ala Ala Thr Ala Thr Met Ala Asp His His Lys Asp Gln

            20                  25                  30
Val Val Tyr Ser Leu Gly Glu Arg Cys Gln Pro Gly Met Gly Tyr Pro
        35                  40                  45
Met Tyr Ser Leu Pro Arg Cys Arg Ala Val Val Lys Arg Gln Cys Val
    50                  55                  60
Gly His Gly Ala Pro Gly Gly Ala Val Asp Glu Gln Leu Arg Gln Asp
65                  70                  75                  80
Cys Cys Arg Gln Leu Ala Ala Val Asp Asp Ser Trp Cys Arg Cys Ser
                85                  90                  95
Ala Leu Asn His Met Val Gly Gly Ile Tyr Arg Glu Leu Gly Ala Thr
            100                 105                 110
Asp Val Gly His Pro Met Ala Glu Val Phe Pro Gly Cys Arg Arg Gly
        115                 120                 125
Asp Leu Glu Arg Ala Ala Ala Ser Leu Pro Ala Phe Cys Asn Val Asp
    130                 135                 140
Ile Pro Asn Gly Thr Gly Gly Val Cys Tyr Trp Leu Gly Tyr Pro Arg
145                 150                 155                 160
Thr Pro Arg Thr Gly His
                165

<210> 49
<211> 8
<212> PRT
<213> Rice
<220> Rice allergen
<222> 96...106
<400> 49
Leu Gly Gly Ile Tyr Xaa Glu Leu
1               5

<210> 50
<211> 8
<212> PRT

<213> Rice
<220> Rice seed allergen RA14
<222> 101...108
<400> 50
Val Gly Gly Ile Tyr Xaa Glu Leu
 1               5

<210> 51
<211> 7
<212> PRT
<213> Homo sapiens
<220> Zinc finger protein 300
<222> 129...135
<400> 51
Gln Xaa Asp Gly Gln Xaa Gln
 1           5

## Description

### TECHNICAL FIELD

The present invention relates to examination reagents useful for the diagnosis of Crohn's disease and to active ingredients thereof. In addition, the invention relates to a method of examining Crohn's disease which can be conveniently carried out using a biological sample, e.g. blood, as the examination sample.

### BACKGROUND TECHNOLOGY

Crohn's disease is acknowledged to be a local inflammatory lesion arising from an abnormal immunologic response. The diagnosis of Crohn's disease has so far been made comprehensively based on clinical symptoms, roentgenography, endoscopic or pathological examination, and so on. However, these methods not only require experience and skill in judgment but also annoy the patient physically and mentally. For this reason, there has been a standing demand for a method by which Crohn's disease may be conveniently and accurately diagnosed.

While the etiology of Crohn's disease remains yet to be elucidated, its relationship to dietary antigens has been pointed out. In fact, it is reported that certain antibodies such as anti-baker's yeast antibody and anti-swine amylase antibody are specifically increased in the sera of patients with Crohn's disease. Based on these findings, methods for diagnosis of Crohn's disease have recently been proposed which involve detecting those antibodies which are specifically present in patients with Crohn's disease, for example anti-baker's yeast antibody (Matsumoto, T. et al.: "Significance of determining serum anti-*Saccharomyces cerevisiae* antibody in inflammatory enteral disease", 1998 Report of Refractory Inflammatory Enteral Disorder Investigation & Study Group; Main J. et al., BMJ, 1988 Oct 29, 297 (6656) 1105-6; Barnes RM. et al., Int. Arch. Allergy Appl. Immunol. 1990, 92(1):9-15; Giaffer MH. et al., Gut. 1992 Aug, 33(8), 1071-5; Sendid B. et al., Clin. Diagn. Lab. Immunol. 1996 Mar, 3(2), 219-26; Quinton JF. et al., Gut. 1998 Jun, 42(6) 788-91), anti-swine amylase antibody (Tozawa, T. et al.: "Anti-swine amylase antibody in the blood of patients with Crohn's disease-studies by ELISA", 1998 Report of Refractory Inflammatory Enteral Disorder Investigation & Study Group; Japanese published un-examination application H11-190734), anti-*M. paratuberculosis*-derived protein antibody (Suenaga, K. et al., Dig. Dis. Sci., 1999, Jun, 44(6), 1202-7; Kreuzpaintner, G. et al., Gut. 1995 Sep, 37(3), 361-6; Oudkerk Pool M. et al., J. Clin. Pathol., 1995 Apr, 48(4), 346-50), or anti-neutrophile antibody (Targan, S. et al., Gastroenterology, 96, A505, 1989), or anti-small intestinal antibody (Bagchi, S. et al.: Clin. Exp. Immuno., 55, 44-48, 1984).

Meanwhile, rice allergen proteins have been isolated as main antigens for IgE in patients with rice allergy and, based on DNA and amino acid sequences, these are known to be alpha-amylase/trypsin inhibitors (Izumi, H. et al., FEBS Lett. 1992 May, 18:302(3), 213-6; Nakamura, R. et al.: Biosci. Biotechnol. Biochem., 1996 Aug, 60(8), 1215-21). Although the relationship of this disease with dietary antigens, e.g. baker's yeast and swine amylase, as self-antigens specific to Crohn's disease has been pointed out as mentioned above, there is no report available as of this day about the relationship to said rice allergen proteins.

Moreover, vacuolar H⁺ transport ATPase is a H⁺ pump which is present in the organelle belonging to the central vacuolar system and regulates the internal milieu of the organelle to the acidic side and it is known that the acidic pH established thereby is closely linked to many vital phenomena inclusive of the dynamic process of the membrane, such as the concentration of neurotransmitters and ions and the decomposition of proteins (Seikagaku (Biochemistry), 65, 6, 1993 June, 413-436). However, its detailed functions *in vivo* remain to be fully elucidated. Furthermore, the human nuclear protein (Homo sapiens kruppel-like zinc finger protein 300 (ZNF300)) is a protein having a zinc-finger domain and, therefore, is suspected to be involved in the control of gene expression within the nucleus but its functions remain unknown. There is no report about this human nuclear protein; all that is known is that its amino acid sequence and the corresponding base sequence have been registered on databases (Gou D.-MET et al., Submitted (28-JUN-2001) to the EMBL/GenBank/DDBJ databases).

These proteins are expected to find application in new drug development and health care as their biological functions are more than more elucidated, but there is no report suggestive of the functions as yet, nor is there a report pointing to the possible relationship of any of such proteins to Crohn's disease.

### DISCLOSURE OF INVENTION

The present invention has for its object to provide examination reagents useful for specific detection Crohn's disease and active ingredients for such reagents. The invention has for its further object to provide an examination method for diagnosing Crohn's disease which can be performed on a biological sample from the subject.

In the course of the intensive research for accomplishing the above objects, the inventors of the present invention found that certain peptides have the property to specifically recognize and bind certain antibodies which are specifically present in patients with Crohn's disease and confirmed that by using these peptides each independently or in a combination of two or more species it is possible to simply and accurately detect Crohn's disease in the subject examined. In addition, the inventors found in the course of the above research that in patients with Crohn's disease an antibody recognizing a vacuolar H⁺-transport ATPase (particularly its subunit E), a rice allergen protein, or a human nuclear protein (Homo sapiens kruppel-like zinc finger protein 300; ZNF300) is specifically present. The inventors were convinced that these proteins are acting as specific self-antigens in Crohn's disease and that Crohn's disease could be accurately diagnosed by detecting the presence/absence of such antibodies in subjects.

The present invention has been developed on the basis of the above findings.

The first aspect of the present invention is concerned with the following Crohn's disease antibody-binding peptides (1) to (9) which can be effectively utilized in the examination of Crohn's disease:
(1) The following Crohn's disease antibody-binding peptide (a) or (b):
   (a) a peptide consisting of an amino acid sequence selected from among the amino acid sequences represented by SEQ ID NOS:1 to 4
   (b) a peptide consisting of a modified amino acid sequence derived from the above-mentioned amino acid sequence (a) by substitution, deletion or addition of one or several amino acids and capable of binding to Crohn's disease antibody.
(2) A Crohn's disease antibody-binding peptide according to paragraph (1) wherein the peptide defined in the above paragraph (b) is a peptide partially comprising an amino acid sequence represented by any of SEQ ID NOS: 1 to 4 and SEQ ID NO:7.
(3) A Crohn's disease antibody-binding peptide according to paragraph (1) wherein the peptide consisting of a modified amino acid sequence derived from the amino acid sequence of SEQ ID NO:1 by substitution, deletion, or addition of one or several amino acids and capable of binding to Crohn's disease antibody is one consisting of the amino acid sequence represented by any of SEQ ID NOS:5 to 14.
(4) A Crohn's disease antibody-binding peptide according to paragraph (1), wherein the peptide consisting of a modified amino acid sequence derived from the amino acid sequence of SEQ ID NO:1 by substitution, deletion, or addition of one or several amino acids and capable of binding to Crohn's disease antibody is a 6 to 226-residue peptide at least comprising the amino acid sequence of LIAQQM.
(5) A Crohn's disease antibody-binding peptide according to paragraph (1), wherein the peptide consisting of a modified amino acid sequence derived from the amino acid sequence of SEQ ID NO:2 by substitution, deletion, or addition of one or several amino acids and capable of binding to Crohn's disease antibody is one consisting of an amino acid sequence represented by any of SEQ ID NOS:15 to 19.
(6) A Crohn's disease antibody-binding peptide according to paragraph (1), wherein the peptide consisting of a modified amino acid sequence derived from the amino acid sequence of SEQ ID NO:3 by substitution, deletion, or addition of one or several amino acids and capable of binding to Crohn's disease antibody is one consisting of an amino acid sequence represented by any of SEQ ID NOS:20 to 32.
(7) A Crohn's disease antibody-binding peptide according to paragraph (1), wherein the peptide consisting of a modified amino acid sequence derived from the amino acid sequence of SEQ ID NO:3 by substitution, deletion, or addition of one or several amino acids and capable of binding to Crohn's disease antibody is a 7 to 604-residue peptide at least comprising the amino acid sequence represented by SEQ ID NO:51.
(8) A Crohn's disease antibody-binding peptide according to paragraph (1), wherein the peptide consisting of a modified amino acid sequence derived from the amino acid sequence of SEQ ID NO:4 by substitution, deletion, or addition of one or several amino acids and capable of binding to Crohn's disease antibody is one consisting of the amino acid sequence represented by any of SEQ ID NOS:33 to 48.
(9) A Crohn's disease antibody-binding peptide according to paragraph (1), wherein the peptide consisting of a modified amino acid sequence derived from the amino acid sequence of SEQ ID NO:4 by substitution, deletion, or addition of one or several amino acids and capable of binding to Crohn's disease antibody is an 8 to 165-residue peptide at least comprising the amino acid sequence of L(V)GGIYXD(E)L (X represents an arbitrary amino acid residue).
   The Crohn's disease antibody-binding peptide according to the present invention may be one having a plurality of sequences selected from among those of the peptides set forth in the above paragraphs (1) to (9) within each molecule.
   As peptides having such structures, the invention provides the branched multiple antigenic peptides defined in the following paragraphs (10) and (11):
(10) A branched multiple antigenic peptide containing the amino acid sequence of the following:
   (a) a peptide consisting of an amino acid sequence selected from among the amino acid sequences represented by SEQ ID NOS:1 to 4 or
   (b) a peptide consisting of a modified amino acid sequence derived from the amino acid sequence set forth in paragraph (a) by substitution, deletion or addition of one or several amino acids and capable of binding to Crohn's disease antibody
      in a plurality of units, which may be the same or different, as branched chain sequences within each molecule.
(11) A branched multiple antigenic peptide according to paragraph (10), which comprises as branched chain sequences the amino acid sequences of two or more dissimilar Crohn's disease antibody-binding peptides selected from at least two of the following groups: (i) a peptide consisting of the amino acid sequence represented by SEQ ID NO:1 and its equivalent, (ii) a peptide consisting of the amino acid sequence represented by SEQ ID NO:2 and its equivalent, (iii) a peptide consisting of the amino acid sequence represented by SEQ ID NO:3 and its equivalent, and (iv) a peptide consisting of the amino acid sequence represented by SEQ ID NO:4 and its equivalent.
   The term 'equivalent' as used herein means any peptide consisting of a modified amino acid sequence derived from the amino acid sequence represented by one of SEQ ID NOS:1 to 4 by substitution, deletion or addition of one or several amino acids and capable of binding to Crohn's disease antibody (the same applies to (13) below).
   The second aspect of the present invention is concerned with examination reagents for Crohn's disease and a reagent kit comprising the same as set forth in the following paragraphs (12) to (15), which are useful for diagnosis of Crohn's disease:
(12) An examination reagent for Crohn's disease which comprises as an active ingredient at least one member selected from the Crohn's disease antibody-binding peptides defined the above paragraphs (1) to (9); the branched multiple antigenic peptide defined in the above paragraph (10); and the complex of subunit E of human vacuolar H⁺-transport ATPase with at least one of the other subunits selected from subunit A, subunit B, subunit C, subunit D, 115 kDa subunit, 39 kDa subunit, 20 kDa subunit, and 16 kDa subunit.
(13) An examination reagent for Crohn's disease as set forth in paragraph (12), wherein said Crohn's disease antibody-binding peptide comprises two or more dissimilar Crohn's disease antibody-binding peptides selected from at least two of the following groups: (i) a peptide consisting of the amino acid sequence represented by SEQ ID NO:1 and its equivalent, (ii) a peptide consisting of the amino acid sequence represented by SEQ ID NO:2 and its equivalent, (iii) a peptide consisting of the amino acid sequence represented by SEQ ID NO:3 and its equivalent, and (iv) a peptide consisting of the amino acid sequence represented by SEQ ID NO:4 and its equivalent, and said branched multiple antigenic peptide is the peptide set forth in paragraph (11).
(14) An examination kit for Crohn's disease which comprises the examination reagent set forth in paragraph (12) or (13) as the antigenic substance for binding Crohn's disease antibody.
(15) An examination kit according to paragraph (14), comprising an anti-human IgG antibody and the examination reagent set forth in paragraph (12) or (13), optionally together with at least one member selected from a sample diluent, a labeling substance, a support (solid phase), a diluent for anti-human IgG antibody, an enzyme substrate solution, and a reaction stop solution.

The third aspect of the present invention is concerned with examination methods for Crohn's disease as set forth below in paragraphs (A) to (C):
(A) An examination method for Crohn's disease which comprises a step of detecting the presence/absence of an antibody recognizing the human vacuolar H⁺-transport ATPase in a biological sample from a subject.
   The above examination method for Crohn's disease includes the following modes:
   (A-1) The examination method for Crohn's disease as set forth in paragraph (A), wherein said antibody is an antibody which recognizes subunit E of human vacuolar H⁺-transport ATPase.
   (A-2) The examination method for Crohn's disease as set forth in paragraph (A), wherein said antibody is an antibody which recognizes the complex of subunit E of human vacuolar H⁺-transport ATPase with at least one of the other subunits selected from subunit A, subunit B, subunit C, subunit D, 115 kDa subunit, 39 kDa subunit, 20 kDa subunit, and 16 kDa subunit.
   (A-3) The examination method for Crohn's disease as set forth in paragraph (A), wherein said antibody is an antibody which recognizes the 199-212 amino acid region of subunit E of human vacuolar H⁺-transport ATPase.
   (A-4) The examination method for Crohn's disease according to any of (A) to (A-3), comprising a step of using as an antigen, any of a peptide consisting of the amino acid sequence of LIAQQM or'its equivalent, and a branched multiple antigenic peptide containing the amino acid sequence of said peptide or equivalent in a plurality of units, which may be the same or different, as branched chain sequences within each molecule,
      and detecting a complex formed by the antigen-antibody reaction between the said antigen and the antibody recognizing human vacuolar H⁺-transport ATPase.
   (A-5) The examination method for Crohn's disease as set forth in paragraph (A-4), wherein the peptide consisting of the amino acid sequence of LIAQQM or its equivalent is a 6 to 227-residue peptide comprising the amino acid sequence of LIAQQM.
   (A-6) The examination method for Crohn's disease as set forth in (A-4), wherein said equivalent of a peptide comprising the amino acid sequence of LIAQQM is a peptide consisting of an amino acid sequence represented by any of SEQ ID NO:1 and NOS:5 to 14.
   (A-7) The examination method for Crohn's disease according to any of paragraphs (A) to (A-3), comprising a step of detecting a complex of subunit E of human vacuolar H⁺-transport ATPase with at least one of the other subunits selected from the group consisting of subunit A, subunit B, subunit C, subunit D, 115 kDa subunit, 39 kDa subunit, 20 kDa subunit, and 16 kDa subunit.
(B) An examination method for Crohn's disease which comprises a step of detecting the presence/absence of an antibody recognizing a human nuclear protein (Homo sapiens kruppel-like zinc finger protein 300) in a biological sample from a subject.
   The above examination method for Crohn's disease comprises the following modes:
   (B-1) The examination method for Crohn's disease as set forth in paragraph (B), wherein said antibody is an antibody which recognizes the 126-138 amino acid region of the human nuclear protein (Homo sapiens kruppel-like zinc finger protein 300).
   (B-2) The examination method for Crohn's disease as set forth in paragraph (B) or (B-1), comprising a step of using as an antigen, any of a peptide consisting of an amino acid sequence represented by SEQ ID NO:51 or its equivalent, and a branched multiple antigenic peptide containing the amino acid sequence of said peptide or equivalent in a plurality of units, which may be the same or different, as branched chain sequences within each molecule,
      and detecting a complex formed by the antigen-antibody reaction between the said antigen and the antibody recognizing the human nuclear protein (Homo sapiens kruppel-like zinc finger protein 300).
   (B-3) The examination method for Crohn's disease as set forth in paragraph (B-2), wherein the peptide consisting of an amino acid sequence represented by SEQ ID NO:51 or its equivalent is a 7 to 604-residue peptide comprising an amino acid sequence represented by SEQ ID NO:51.
   (B-4) The examination method for Crohn's disease as set forth in paragraph (B-2), wherein said equivalent of the peptide represented by SEQ ID NO:51 is a peptide consisting of an amino acid sequence represented by any of SEQ ID NOS:3 and 21 to 32.
(C) An examination method for Crohn's disease which comprises a step of detecting the presence/absence of an antibody recognizing a rice allergen protein in a biological sample from a subject.
   (C-1) The examination method for Crohn's disease as set forth in paragraph (C), wherein said rice allergen protein belongs to the gene family of alpha-amylase/trypsin inhibitors.
   (C-2) The examination method for Crohn's disease as set forth in any of paragraphs (C) to (C-1), wherein said rice allergen protein is at least one member selected from Rice allergen, Rice seed allergen RA5, Rice allergen RA5B precursor, Rice seed allergen RA14, Rice allergen RA14B precursor, and Rice seed allergen RAG2.
   (C-3) The examination method for Crohn's disease as set forth in any of paragraphs (C) to (C-2), wherein said antibody recognizing the rice allergen protein is an antibody which recognizes the 99-111 amino acid region of Rice seed allergen RA14.
   (C-4) The examination method for Crohn's disease as set forth in any of paragraphs (C) to (C-3), wherein said antibody recognizing the rice allergen protein is an antibody recognizing an amino acid region comprising the amino acid sequence L(V)GGIYREL of the gene family of alpha-amylase/trypsin inhibitors.
   (C-5) The examination method for Crohn's disease as set forth in any of paragraphs (C) to (C-4), comprising a step of using as an antigen, any of a peptide consisting of the amino acid sequence of L(V)GGIYXD(E)L (X represents an arbitrary amino acid residue which may be the same or different) or its equivalent, and a branched multiple antigenic peptide containing the amino acid sequence of said peptide or equivalent in a plurality of units, which may be the same or different, as branched chain sequences within each molecule as the antigen,
      and detecting a complex formed by the antigen-antibody reaction between the said antigen and the antibody recognizing the rice allergen protein.
   (C-6) The examination method for Crohn's disease as set forth in paragraph (C-5), wherein the peptide consisting of the amino acid sequence of L(V)GGIYXD(E)L (X represents an arbitrary amino acid residue which may be the same or different) or its equivalent is an 8 to 166-residue peptide at least comprising the amino acid sequence of L(V)GGIYXD(E)L (X represents an arbitrary amino acid residue which may be the same or different).
   (C-7) The examination method for Crohn's disease as set forth in paragraph (C-5), wherein said equivalent of the peptide consisting of the amino acid sequence of L(V)GGIYXD(E)L (X represents an arbitrary amino acid residue which may be the same or different) is a peptide consisting of an amino acid sequence represented by any of SEQ ID NOS:4 and 33 to 48.

The present invention further includes the following inventions:
(a) Use of the peptide defined in any of the above paragraphs (1)to(11) as the antigen to be reacted with Crohn's disease antibody in an examination for Crohn's disease.
(b) Use of the peptide defined in any of the above paragraphs (1)to(11) for the manufacture of examination reagents for Crohn's disease.

The representation of amino acids, peptides, base sequences, nucleic acids, etc. by abbreviations in this specification complies with the rules of IUPAC and IUB, "Guidelines for drafting of specifications etc. containing base sequences or amino acid sequences" (edited by the Patent Office of Japan), and the nomenclature in routine use in this field of art. It should also be understood that the "peptide" in the present invention includes an oligopeptide consisting of not more than 10 amino acids and a polypeptide consisting of more than 10 amino acids.

The "Crohn's disease antibody" is a Crohn's disease-specific antibody which is specifically produced in the body which has contacted Crohn's disease. In the context of the invention, this term broadly means the antibody characteristic of patients with Crohn's disease which is specifically found in such patients regardless of the kind of causative antigen and whether recognized or not. More particularly, it means the antibody which is specifically found in patients with Crohn's disease at least in contrast with healthy subjects, patients with ulcerative colitis, patients with any other autoimmune disease, patients with duodenal ulcer, and patients with gastric ulcer. Incidentally, this Crohn's disease antibody is usually contained in various biological samples from patients with Crohn's disease, such as blood (serum, plasma), urine, sweat, saliva, seminal fluid, and spinal fluid.

### BRIEF DESCRIPTION OF DRAWINGS

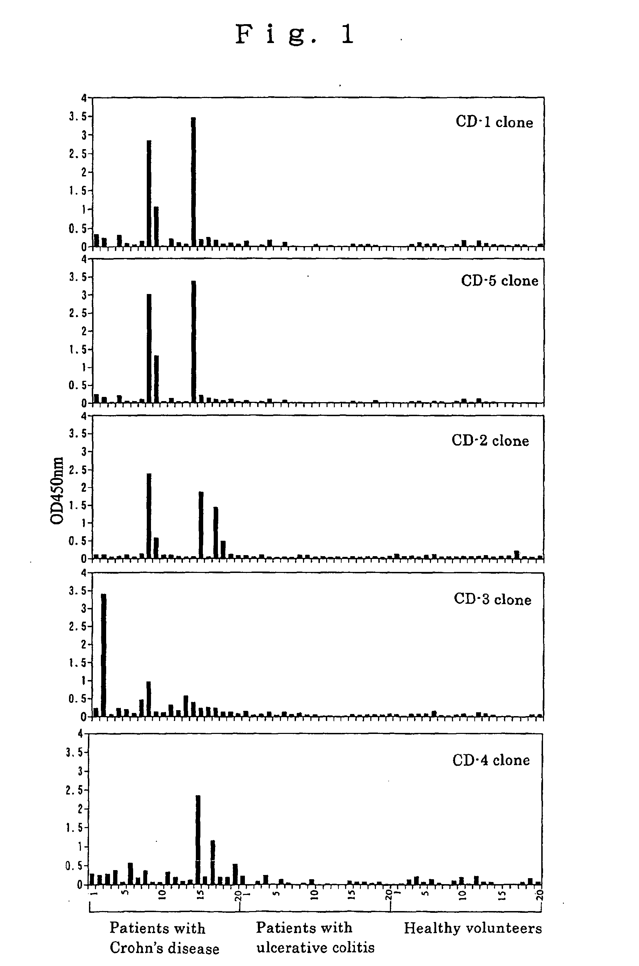

Fig. 1 is a diagram showing the results of Example 1 (2). Thus, using 5 clones (CD-1, CD-2, CD-3, CD-4, CD-5) selected from the specificity to the sera of patients with Crohn's disease, the reactivity to various serum samples (sera of patients with Crohn's disease, sera of patients with ulcerative colitis, and sera of healthy volunteers) was investigated by ELISA and the results are indicated.

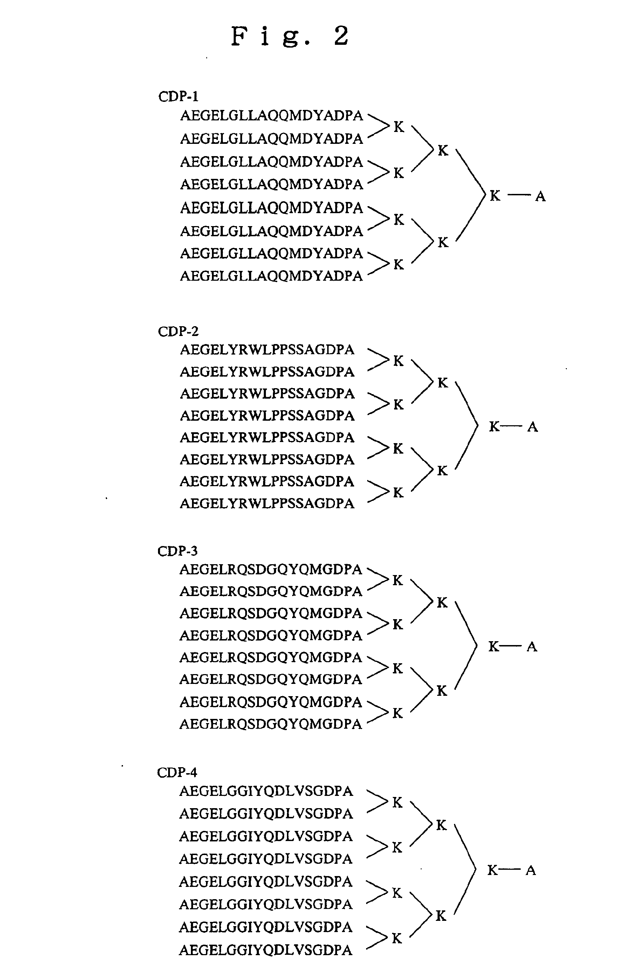

Fig. 2 is a diagram showing the structures of MAP peptides (MAP peptides of CDP-1, CDP-2, CDP-3, and CDP-4).

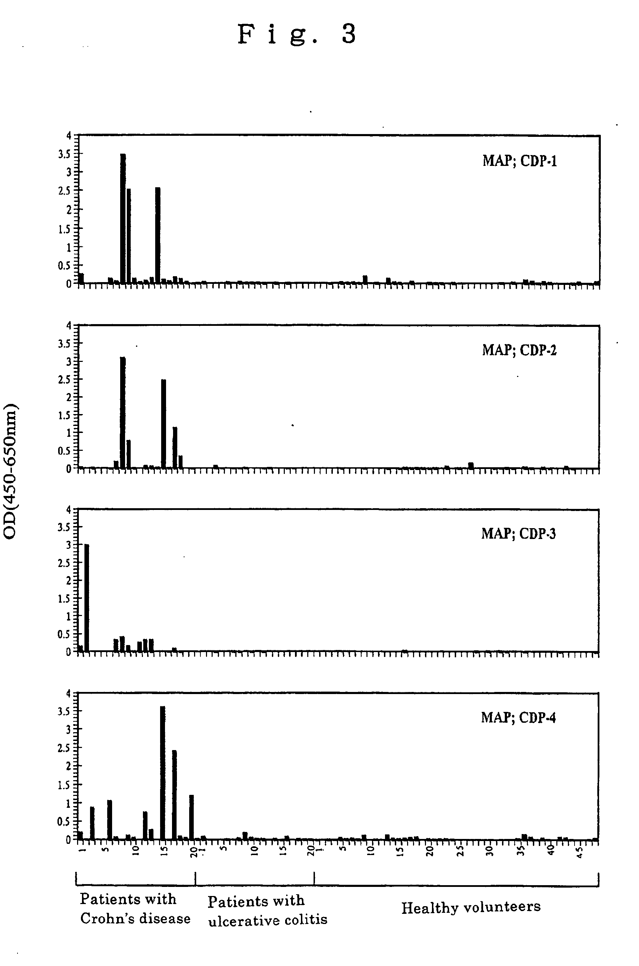

Fig. 3 is a diagram showing the results of Example 2 (1). Thus, the reactivity of various serum samples (sera of patients with Crohn's disease, sera of patients with ulcerative colitis, sera of healthy volunteers) to each of MAP peptides (MAP peptides of CDP-1, CDP-2, CDP-3, and CDP-4) was investigated by ELISA and the results were indicated.

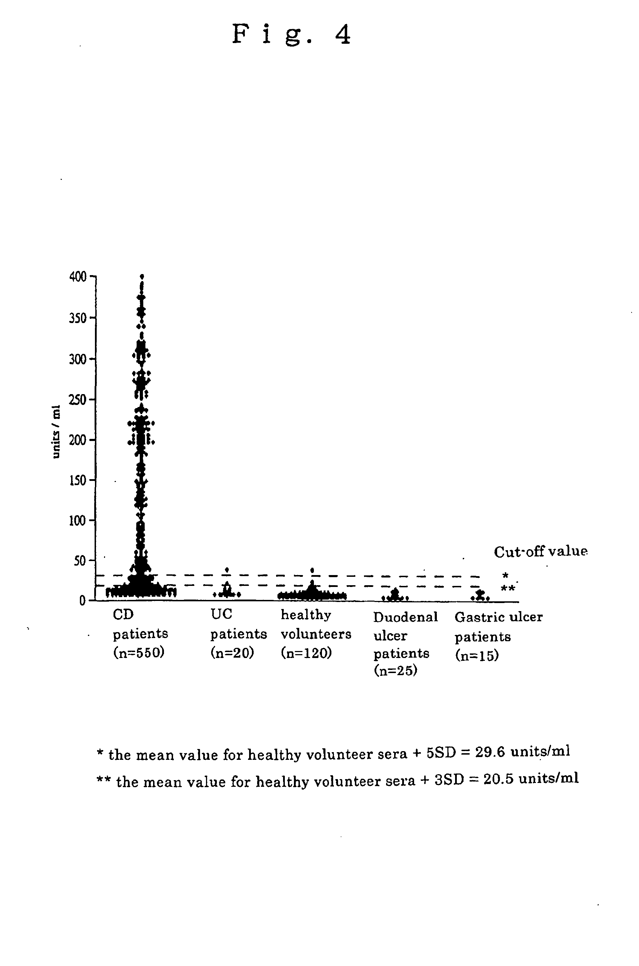

Fig. 4 is a diagram showing the results of Example 2 (2). Thus, using a mixed antigen plate, the reactivity of 550 serum samples from Crohn's disease (CD) patients, 20 serum samples from ulcerative colitis (UC) patients, 120 serum samples from healthy volunteers, 25 serum samples from duodenal ulcer patients, and 15 serum samples from gastric ulcer patients to the mixed MAP peptide was investigated by ELISA and the results were indicated.

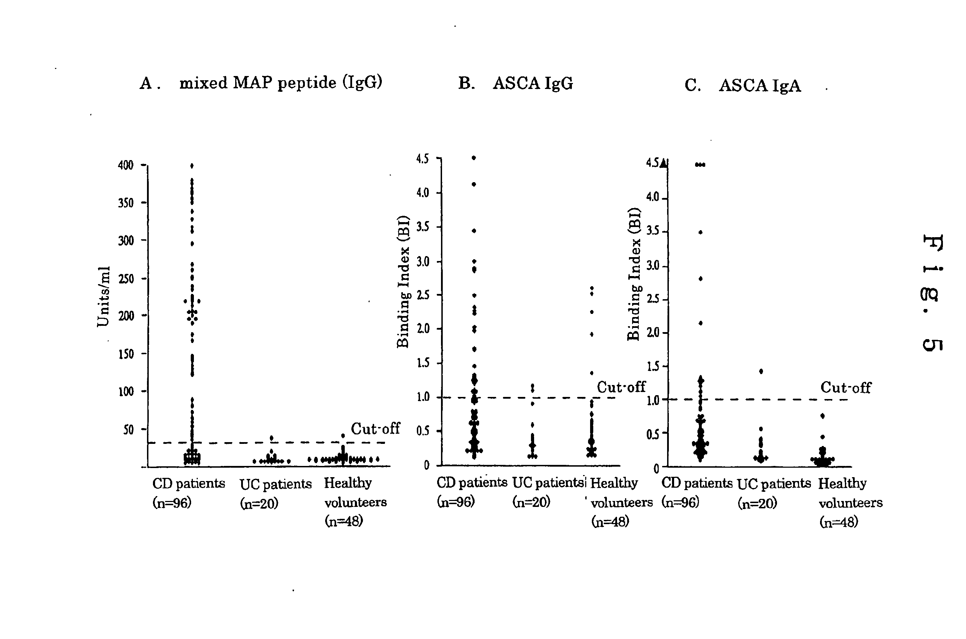

Fig. 5 is a diagrammatic representation of the results of Example 2 (3). More particularly, Fig. A is a diagram showing the reactivity of various serum samples (sera of Crohn's disease (CD) patients, sera of ulcerative colitis (UC) patients, and sera of healthy volunteers) to the mixed MAP peptide. Figs. B and C show the reactivity of various serum samples (sera of Crohn's disease (CD) patients, sera of ulcerative colitis (UC) patients, and sera of healthy volunteers) to baker's yeast used as the antigen in lieu of the mixed MAP peptide. Commercial anti-*Saccharomyces cerevisiae* antibodies (ASCA: ASCA IgG and ASCA IgA) detection kits were used. ASCA IgG and ASCA IgA were used in Fig. B and C, respectively.

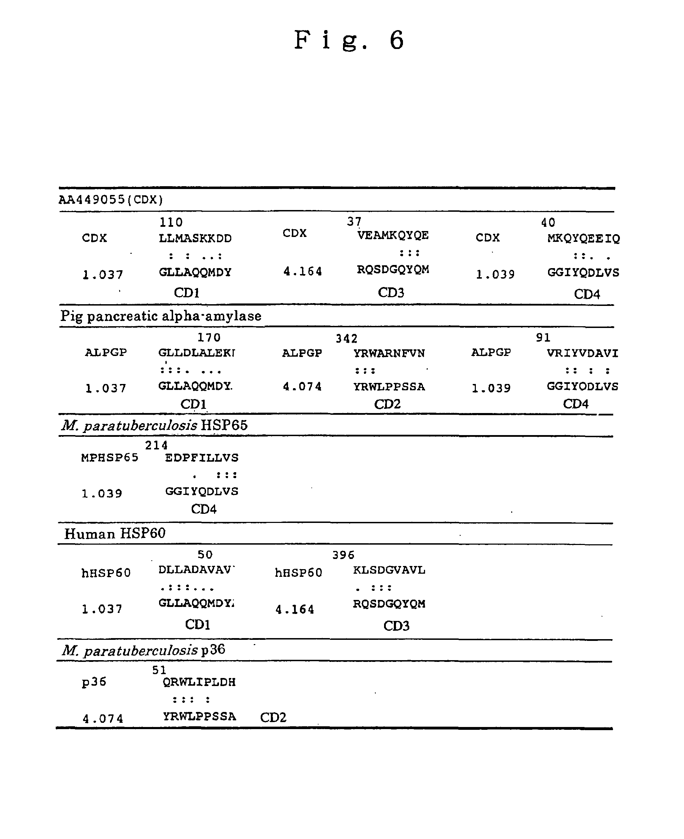

Fig. 6 is a diagram showing the homology in amino acid sequence of peptides specific to patients with Crohn's disease (Crohn's disease antibody-binding peptides: CD1 peptide, CD2 peptide, CD3 peptide, and CD4 peptide) with proteins reportedly related to Crohn's disease [CDX, pig pancreatic alpha-amylase, *M. paratuberculosis* HSP65, human HSP60, and *M. paratuberculosis* p36]. On the diagram, ":" indicates an agreement in the amino acid and "·" indicates a similarity in the amino acid.

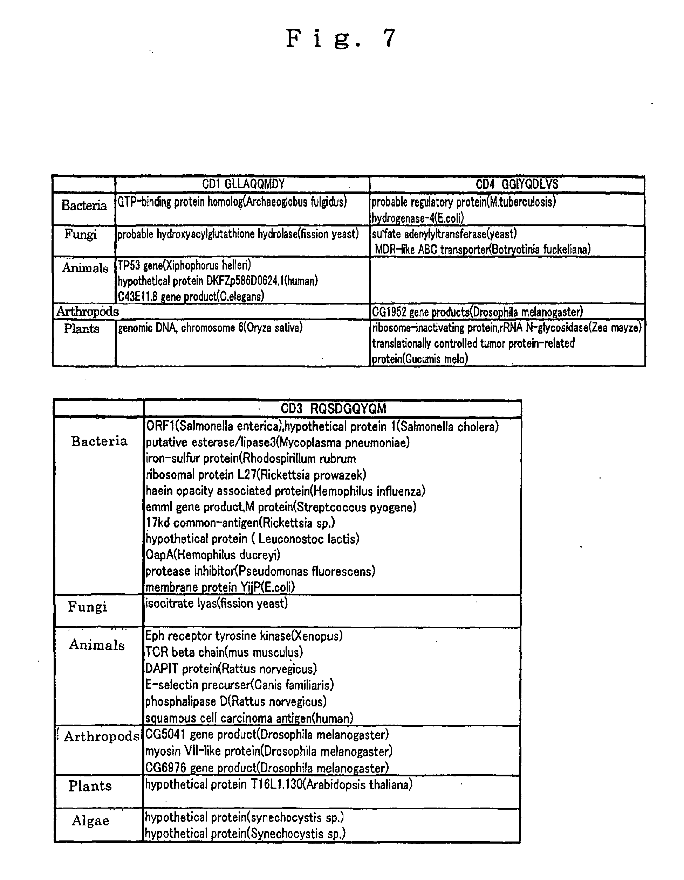

Fig. 7 is a diagram showing the proteins derived from various organisms (bacteria, fungi, animals, arthropods, plants, and algae) and having homology in amino acid sequence with peptides specific to patients with Crohn's disease (Crohn's disease antibody-binding peptides: CD1 peptide, CD3 peptide, and CD4 peptide), as obtained by homology searches through protein databases.

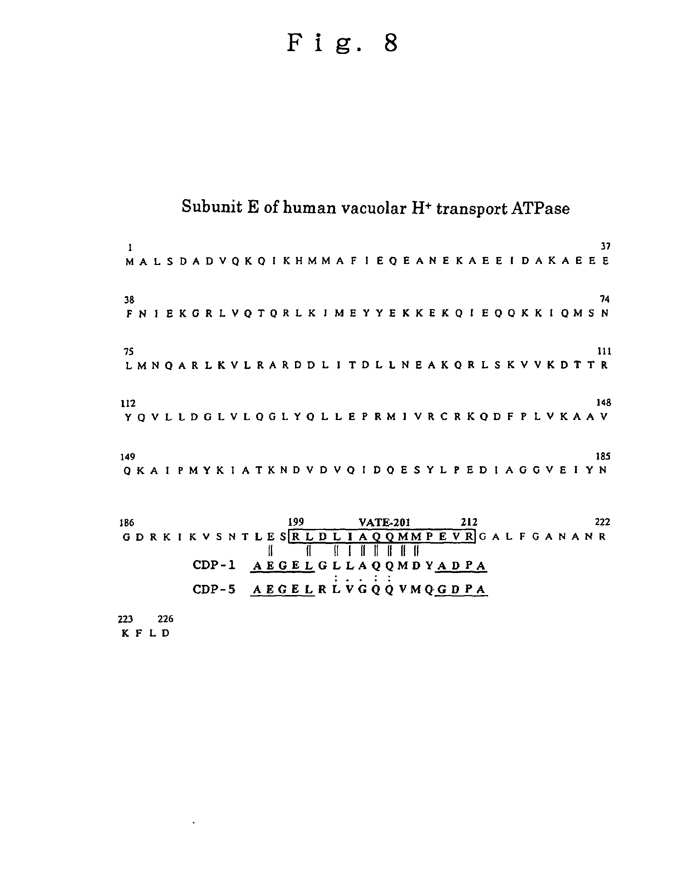

Fig. 8 is a diagram showing the homology of the amino acid sequence of subunit E of human vacuolar H⁺ transport ATPase with the amino acid sequences of equivalents of CD1 peptide (CDP-1 peptide, CDP-5 peptide). On the diagram, "¦" indicates an agreement in the amino acid sequence between V-ATPase subunit E and both or either one of CDP-1 and CDP-5 peptides and "¦" indicates a similarity in the amino acid sequence between V-ATPase subunit E and both of CDP-1 and CDP-5 peptides. Moreover, ":" indicates an agreement in the amino acid between CDP-1 and CDP-5 and "·" indicates a similarity in the amino acid between CDP-1 and CDP-5 peptides. The underscored sequence is the amino acid sequence derived from phage pVIII protein.

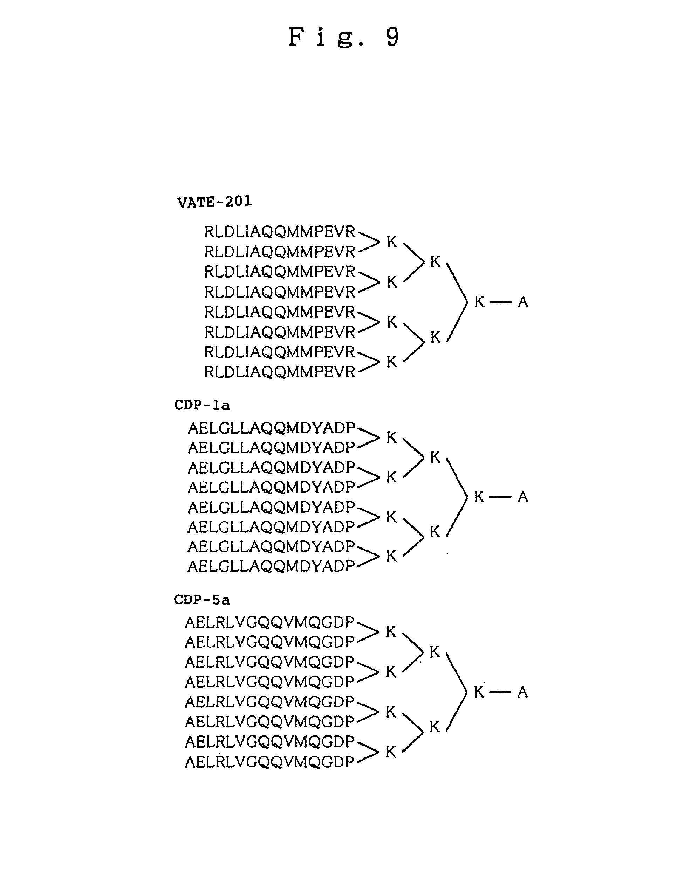

Fig. 9 is a diagram showing the structures of the MAP peptides of VATE-201, CDP-1a, and CDP-5a, respectively.

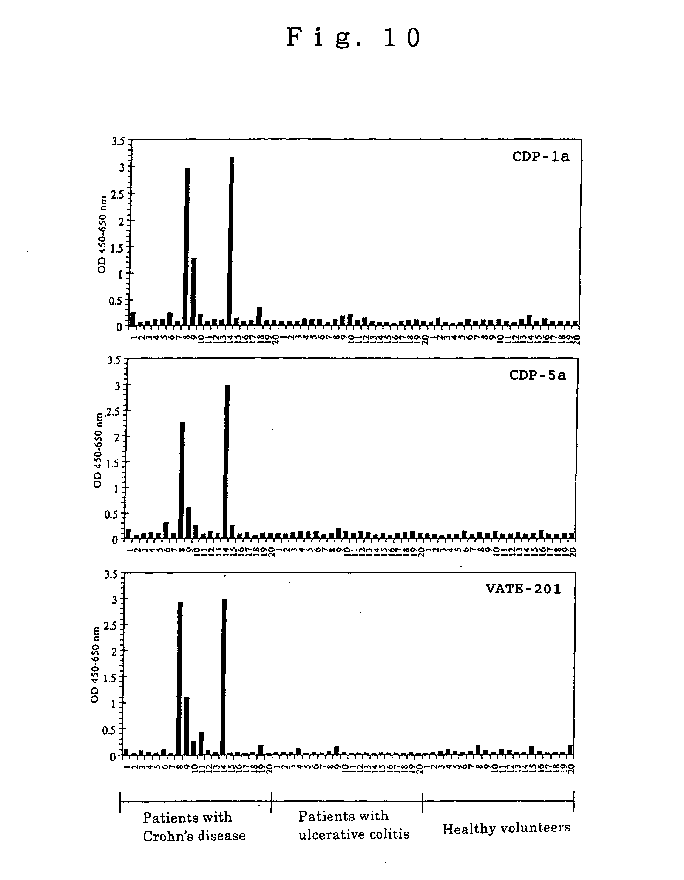

Fig. 10 is a diagram showing the results of Example 4. Thus, the reactivity of CDP-1a peptide, CDP-5a peptide, and the peptide (VATE-201 peptide) derived from subunit E of human vacuolar H⁺ transport ATPase to various serum samples (sera of Crohn's disease patients, sera of ulcerative colitis patients, and sera of healthy volunteers) was investigated by ELISA and the results are shown.

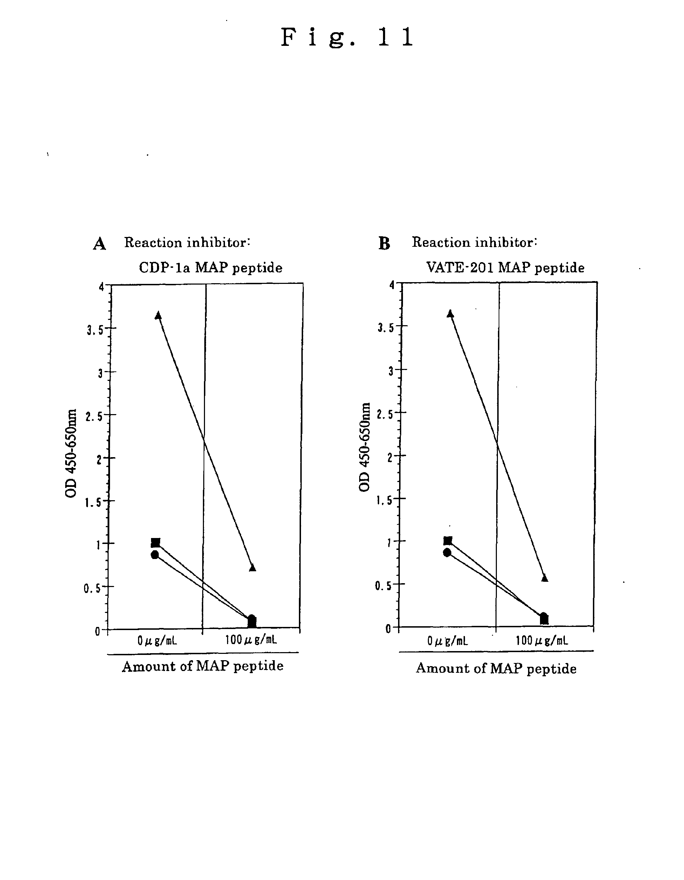

Fig. 11 is a diagram showing the results of Example 5. Represented are the results of an experiment in which the reactivity of Crohn's disease patient serum sample No. 8, No. 9, and No. 14 to the CDP-1a MAP plate was inhibited with the CDP-1a MAP peptide antigen (Fig. A) or VATE-201 MAP peptide antigen (Fig. B). On the diagram, -¦-indicates Crohn's disease patient serum No. 8, -?-indicates Crohn's disease patient serum No. 9, and -?-indicates Crohn's disease patient serum No. 14.

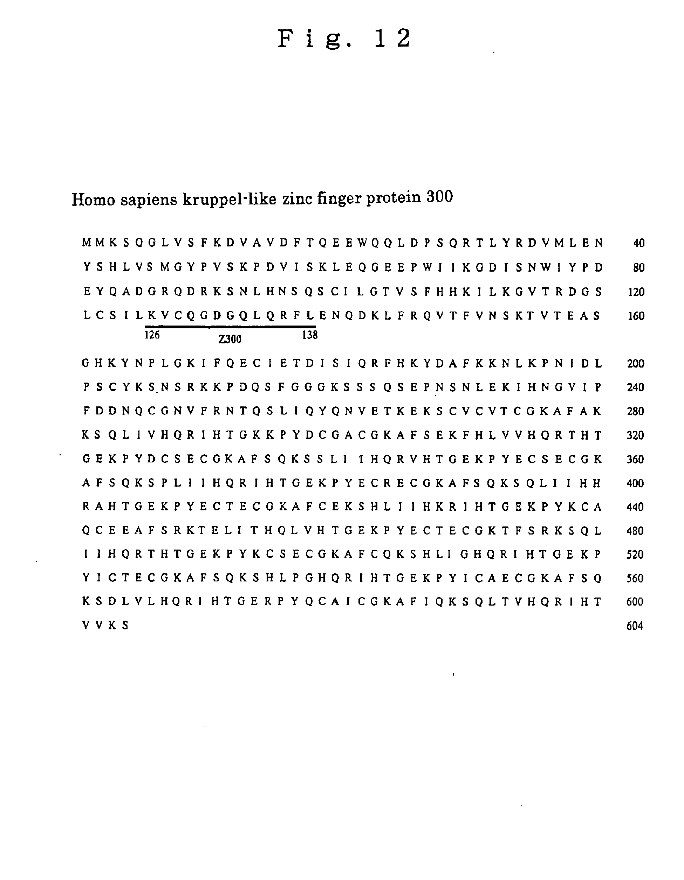

Fig. 12 is a diagram showing the amino acid sequence of Homo sapiens kruppel-like zinc finger protein 300 and the location of Z300 peptide, which has an amino acid sequence corresponding to the 126-138 amino acid region thereof.

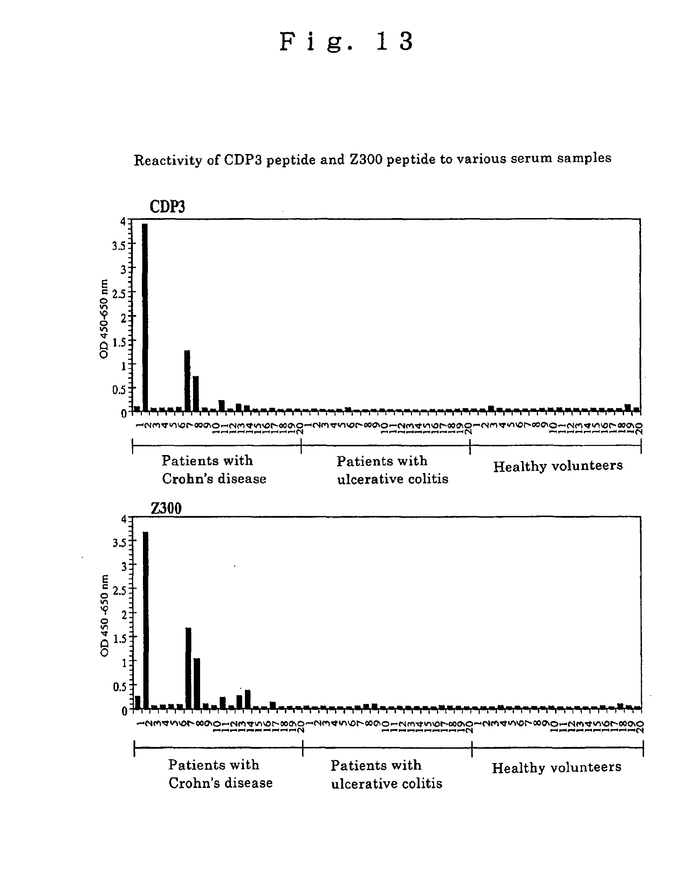

Fig. 13 is a diagram showing the results of Example 6 (4). Thus, the reactivity of CDP3 peptide (top) and Z300 peptide (bottom) to various serum samples (Crohn's disease patient sera, ulcerative colitis patient sera and healthy volunteer sera) was investigated by ELISA and the results are shown.

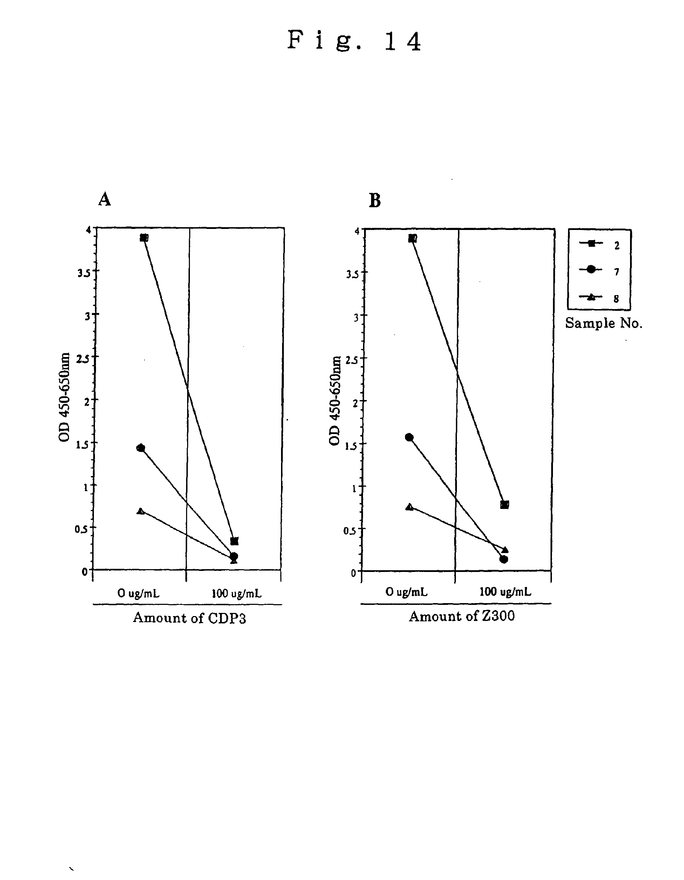

Fig. 14 is a diagram showing the results of Example 6 (5). Represented are the results of an experiment in which the reactivity of Crohn's disease patient serum sample No. 2, No. 7 and No. 8 to the CDP3 MAP plate was inhibited with CDP3 MAP peptide antigen (Fig. A) or Z300 MAP peptide antigen (Fig. B). On the diagram, -¦- indicates Crohn's disease patient serum No. 2, -?- indicates Crohn's disease patient serum No. 7, and -?- indicates Crohn's disease patient serum No. 8.

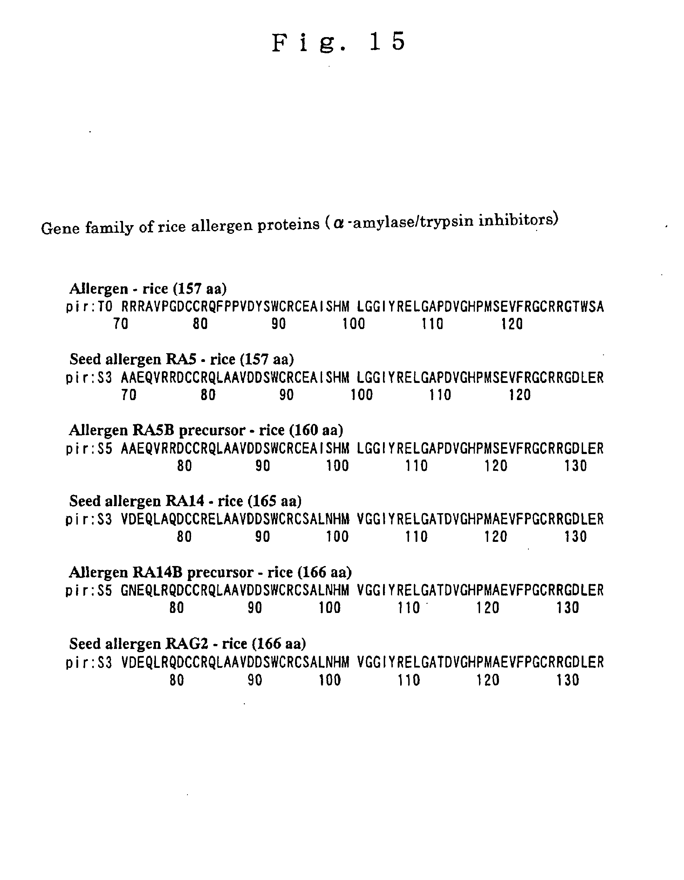

Fig. 15 is a diagram comparing the homology in amino acid sequences of the 95-110 amino acid regions among Rice allergen, Rice seed allergen RA5, Rice allergen RA5B precursor, Rice seed allergen RA14, Rice allergen RA14B precursor, and Rice seed allergen RAG2, which belong to the gene family of rice allergen proteins (a-amylase/trypsin inhibitors).

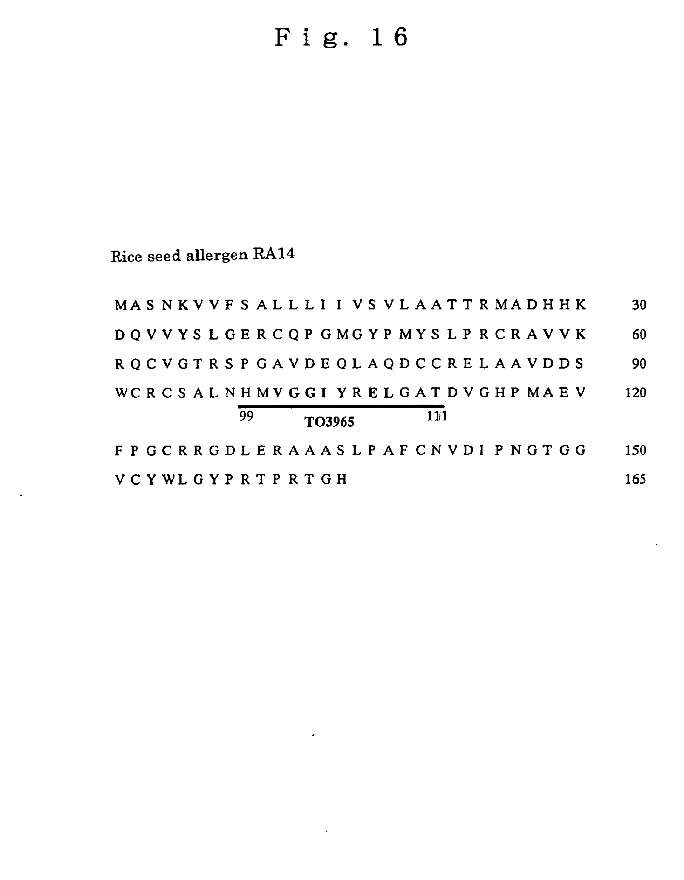

Fig. 16 is a diagram showing the amino acid sequence of a rice allergen protein (Rice seed allergen RA14) and the location of TO3965 peptide, which has an amino acid sequence corresponding to the 99-111 amino acid region thereof.

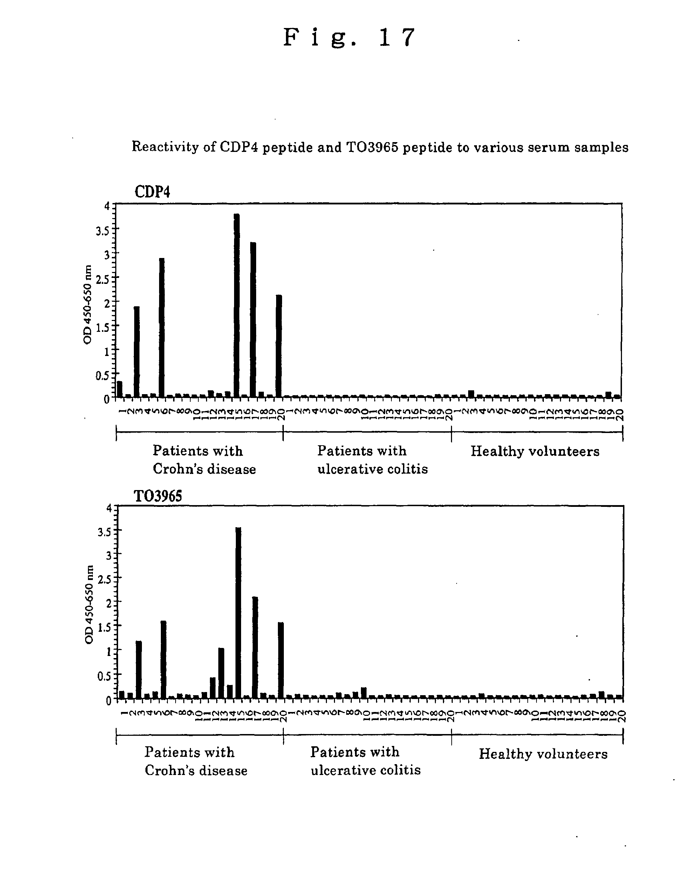

Fig. 17 shows the results of Example 7 (4). Thus, the reactivity of CDP4 peptide (top) and TO3965 peptide (bottom) to various serum samples (Crohn's disease patient sera, ulcerative colitis patient sera, and healthy volunteer sera) was investigated by ELISA and the results are shown.

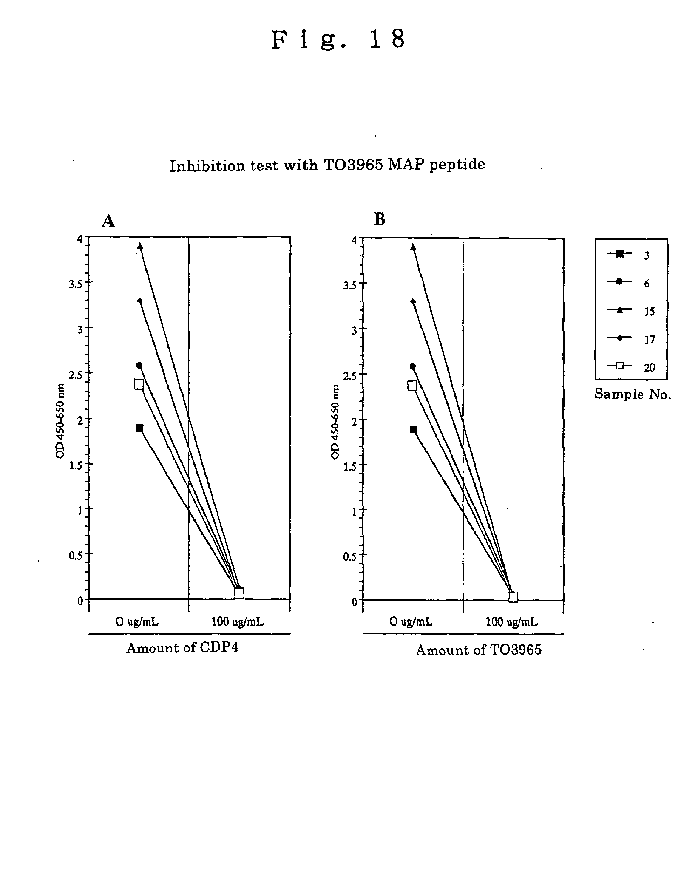

Fig. 18 is a diagram showing the results of Example 7 (5). Represented are the results of an experiment in which the reactivity of Crohn's disease patient serum sample No. 3, No. 6, No. 15, No. 17 and No. 20 to the CDP4 MAP plate was inhibited with CDP4 MAP peptide antigen (Fig. A) or TO3965 MAP peptide antigen (Fig. B). On the diagram, -¦-indicates Crohn's disease patient serum No. 3, -?-indicates Crohn's disease patient serum No. 6, -?-indicates Crohn's disease patient serum No. 15, -?-indicates Crohn's disease patient serum No. 17, and -?-indicates Crohn's disease patient serum No. 20.

### BEST MODES FOR CARRYING OUT THE INVENTION

### (1) Crohn's disease antibody-binding peptides

The "Crohn's disease antibody-binding peptide (hereinafter referred to briefly as CD-binding peptide)" to which the present invention is directed is a peptide which binds specifically to Crohn's disease antibody, i.e. Crohn's disease-specific antibodies which are specifically found in patients with Crohn's disease.

The CD-binding peptide according to the invention specifically includes but is not limited to a peptide consisting of the amino acid sequence represented by any of SEQ ID NOS:1 to 4 (SEQ ID NO:1 = CD1 peptide, SEQ ID NO:2 = CD2 peptide, SEQ ID NO:3 = CD3 peptide, SEQ ID NO:4 = CD4 peptide). These are invariably characterized by their specific binding affinity for Crohn's disease antibody.

Furthermore, the peptide of the invention encompasses not only the above-mentioned peptide consisting of the amino acid sequence represented by any of SEQ ID NOS:1 to 4 but also a peptide having modified amino acid sequence derived from the amino acid sequence referred to just above by substitution, deletion or addition of one or several amino acids and capable of binding to Crohn's disease antibodies. In the context of the invention, these peptides are sometimes referred to as "equivalents" of the peptide consisting of the amino acid sequence represented by any of SEQ ID NOS:1 to 4.

In this connection, the extent and position, for instance, of said "substitution, deletion or addition" are not particularly restricted insofar as the peptide so modified has the property that, just like the peptide having a amino acid sequences represented by any of SEQ ID NOS:1 to 4 (CD1 peptide, CD2 peptide, CD3 peptide, CD4 peptide), it is capable of binding specifically to Crohn's disease antibody, that is to say it is the equivalent of the unmodified peptide. Modification (mutation) of an amino acid sequence may occur through mutation or post-translational modification, for instance, but can be artificially induced as well. Incidentally, the technology for modification (mutagenesis) of amino acid sequences is well known to those skilled in the art (for example, genetic engineering techniques such as site-directed mutagenesis [Methods in Enzymology, 154, 350, 367-382 (1987); Methods in Enzymology, 100, 468 (1983); Nucleic Acids Res., 12, 9441 (1984); Seminar on Experiments in Biochemistry 1, "Methods for Gene Research-II", edited by Japanese Biochemical Society, p.105 (1986), etc.] or chemical synthesis such as the phosphoric triester method or the phosphoramidite method [J. Am. Chem. Soc., 89, 4801 (1967); J. Am. Chem. Soc., 91, 3350 (1969); Science, 150, 178 (1968); Tetrahedron Lett., 22, 1859 (1981); Tetrahedron Lett., 24, 245 (1983)] can be mentioned).

The present invention encompasses all the modified peptides capable of binding specifically to Crohn's disease antibodies regardless of the means or modes of modification or mutation involved.

The equivalent referred to above can be acquired by screening techniques using display phage libraries (phage display libraries), preferably random peptide display phage libraries. The screening techniques utilizing such phage display libraries are known as the phage display method and constitute a known technology which has heretofore been used for the purpose of identifying ligands binding specifically to various cell surface receptors or epitopes recognizing various antibodies. Regarding methods for constructing such phage display libraries and in vitro screening protocols, the methods of Scott and Smith can be used as references (Scott, J. M. and Smith, G. P., Science, 249, 386-390 (1990); Smith, G. P. and Scott, J. K., Methods in Enzymology, 217, 228-257 (1993)).

The random peptide display phages in said libraries can be utilized for the *in vitro* expression of a large number of peptides (oligopeptides or polypeptides) as subjects of screening for sorting out and identifying peptides capable of biding specifically to Crohn's disease antibodies. Moreover, the phage library to be used may be any known phage library that is routinely used in this method and, to mention a preferred example, it may be a random peptide display phage (filamentous phage) constructed by inserting a random DNA into the phage coat protein pIII gene so as to permit expression of a peptide having a random 15-residue amino acid sequence on the surface of the phage capsid. (Japanese published un-examined patent application No.H10(1998)-237098, Japanese published un-examined patent application No.H10(1998)-237099, Ishikawa, F. & Taki, T.: Saibou Kougaku (Cell Engineering), 16 (2), 1821-1828 (1997), Japanese published un-examined patent application No.2000-253900).

For acquiring the equivalent of any of said CD1 peptide (SEQ ID NO:1), CD2 peptide (SEQ ID NO:2), CD3 peptide (SEQ ID NO:3), and CD4 peptide (SEQ ID NO:4) by the above screening method, the following specific procedures can be mentioned.

First, a random peptide display phage library is constructed by inserting a random DNA sequence into a phargemid vector in such a manner that a peptide having a random amino acid sequence corresponding to said DNA sequence may be expressed on the surface of the phage capsid. This phage library is reacted with the Crohn's disease patient serum antibody (IgG) immobilized on the solid-phase surface, such as a microplate, through an anti-human IgG antibody in advance, and the phage binding specifically to said Crohn's disease patient serum antibody is recovered (biopanning). As the random DNA sequence to be inserted into said phagemid vector, a DNA sequence coding for a modified amino acid sequence derived from the peptide such as CD1 peptide, CD2 peptide, CD3 peptide or CD4 peptide, of which said equivalent is to be acquired, by deletion, substitution or addition of at least one amino acid can be selected.

In this connection, the Crohn's disease patient serum antibody (IgG) to be immobilized on, for example, a microplate is not particularly restricted provided that it has at least an antigen-binding capacity. Thus, it may be the serum obtained from a patient with Crohn's disease as it is or a purified antibody obtained by purifying the serum with protein A or a purified antibody obtained by precipitation with magnesium sulfate solution.

Recovery of the phage bound specifically to Crohn's disease antibody can be achieved by permitting a substance capable of inhibiting the binding of Crohn's disease antibody to the phage to act upon said immobilized antibody. Thus, as said substance is added, the phage bound specifically to the Crohn's disease antibody immobilized on the microplate through antihuman IgG antibody is released or eluted for recovery. By repeating such biopanning a few times, preferably in 2 or 3 rounds, the phage capable of expressing the peptide specifically binding to Crohn's disease antibody can be selected and enriched.

The above substance capable of inhibiting the binding of Crohn's disease antibody to the phage is not particularly restricted but acidic or alkaline solutions, high-concentration salts, urea, and thiocyanogen can be mentioned as examples.

Then, the phage acquired by the above procedure is used to infect *Escherichia coli*, followed by cultivation in large scale, separation and purification to obtain the phage expressing the peptide capable of binding specifically to Crohn's disease antibody. The phage thus obtained is immobilized on a support (solid phase) through an anti-phage antibody and submitted to a screening for a phage capable of binding specifically to Crohn's disease antibody. Specifically this screening is performed by a procedure which comprises reacting the phage obtained by the above procedure with the anti-phage antibody immobilized on an arbitrary support to immobilize it, causing a Crohn's disease patient serum and, as control serum, a healthy volunteer serum or a serum from a patient with an ulcerative colitis, a gastric ulcer or a duodenal ulcer to react with the immobilized phage, and selecting a phage reacting specifically to the Crohn's disease patient serum according to reactivity. From the selected phage, the DNA is isolated by extraction and sequenced, and based on the base sequence, the amino acid sequence is determined. In this manner, the peptide which the selected phage expresses, namely the equivalent (CD-binding peptide) of CD1 peptide, CD2 peptide, CD3 peptide or CD4 peptide which is capable of binding specifically to Crohn's disease antibody can be identified and acquired.

In this connection, the sequencing of the DNA extracted and isolated by the above procedure can be easily carried out by any of the known techniques in the art, for example the dideoxy method [Proc. Natl. Acad. Sci. USA., 74, 5463-5467 (1977)] or Maxam-Gilbert method [Method in Enzymology, 65, 499 (1980)]. Such sequencing can be easily carried out by using a commercial sequencing kit, too.

As examples of the equivalent of CD1 peptide (SEQ ID NO:1) which can be obtained by the above procedure, there can be mentioned the peptides indicated in Table 1, namely CDP-1a peptide (SEQ ID NO:5), CDP-1 peptide (SEQ ID NO:6), CD5 peptide (SEQ ID NO:7), CDP-5a peptide (SEQ ID NO:8), CDP-5 peptide (SEQ ID NO:9), VATE-201c peptide (SEQ ID NO:10), VATE-201 peptide (SEQ ID NO:11), CD1s peptide (SEQ ID NO:12), and CDP-1s peptide (SEQ ID NO:13). Referring to each of these peptides listed in Table 1, the part in common with the amino acid sequence of CD1 peptide is boxed and the resembling amino acid residue is underscored (The same applies to Tables 2 to 4 below).

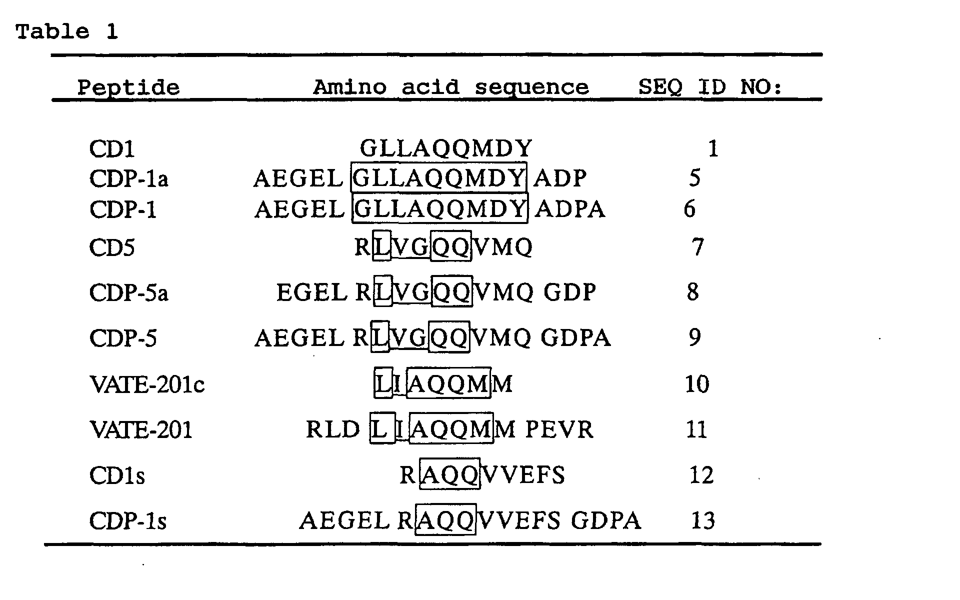

The equivalent of CD2 peptide (SEQ NO:2) includes the peptides listed in Table 2, namely CDP-2 peptide (SEQ ID NO:15), CD2s peptide (SEQ ID NO:16), CDP-2s peptide (SEQ ID NO:17), CD2s1 peptide (SEQ ID NO:18), and CDP-2s1 peptide (SEQ ID NO:19).

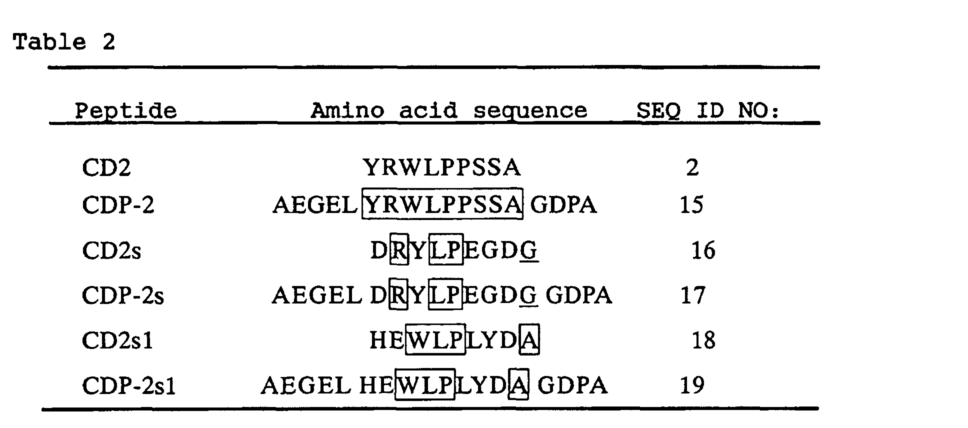

Furthermore, the equivalent of CD3 peptide (SEQ ID NO:3) includes the peptides listed in Table 3, namely CDP-3 peptide (SEQ ID NO:20), CDP3 peptide (SEQ ID NO:21), CDP3-1 peptide (SEQ ID NO:22), CDP3-2 peptide (SEQ ID NO:23), CDP3-3 peptide (SEQ ID NO:24), CDP3-4 peptide (SEQ ID NO:25), CDP3-5 peptide (SEQ ID NO:26), CDP3-6 peptide (SEQ ID NO:27), CDP3-8 peptide (SEQ ID NO:28), CDP3-12 peptide (SEQ ID NO:29), CDP3-14 peptide (SEQ ID NO:30), and Z300 peptide (SEQ ID NO:31).

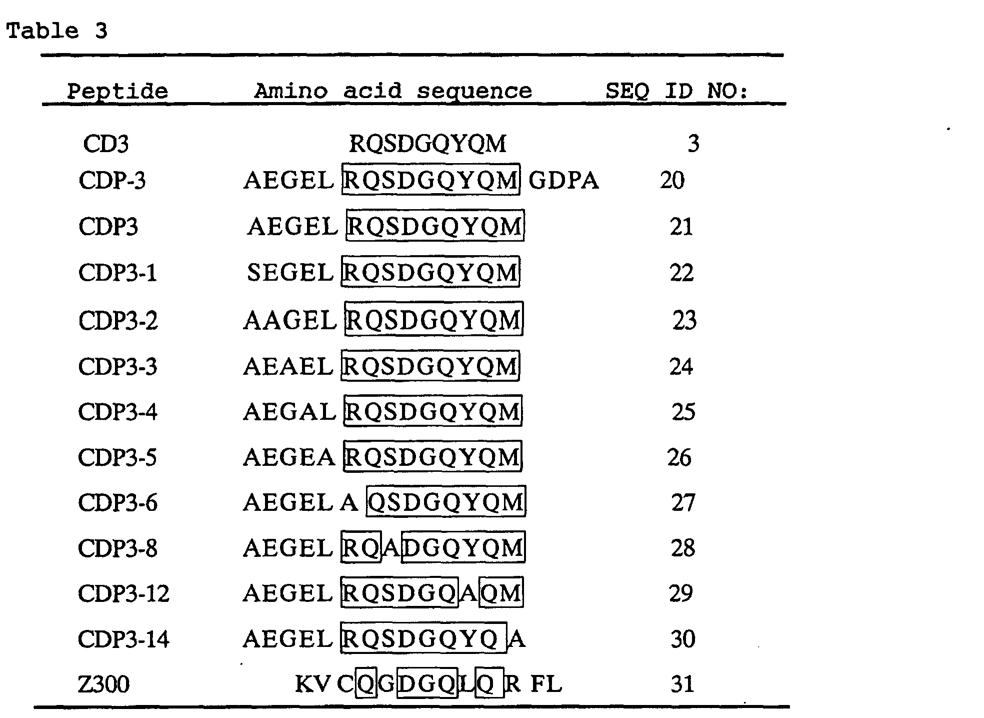

Furthermore, the equivalent of CD4 peptide (SEQ ID NO:4) includes the peptides listed in Table 4, namely CDP-4 peptide (SEQ ID NO:33), CDP4 peptide (SEQ ID NO:34), CDP4-1 peptide (SEQ ID NO:35), CDP4-2 peptide (SEQ ID NO:36), CDP4-3 peptide (SEQ ID NO:37), CDP4-4 peptide (SEQ ID NO:38), CDP4-10 peptide (SEQ ID NO:39), CDP4-13 peptide (SEQ ID NO:40), CDP4-14 peptide (SEQ ID NO:41), and TO3965 peptide (SEQ ID NO:42).

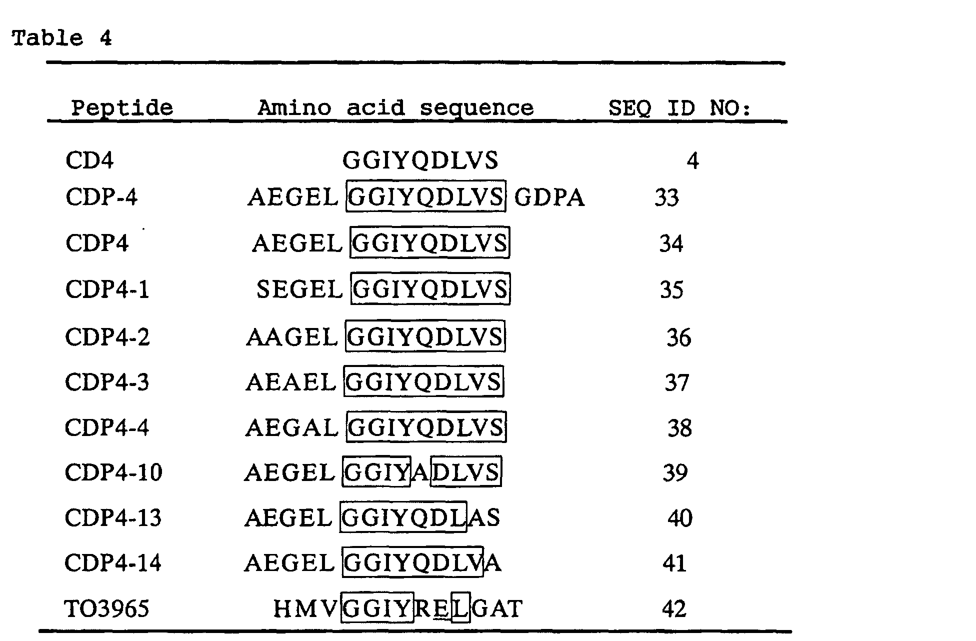

These CD-binding peptides can be produced by the general method of chemical synthesis based on information on the respective amino acid sequences. The method includes ordinary liquid-phase and solid-phase techniques for peptide synthesis. This method of chemical synthesis specifically includes the stepwise elongation method in which the respective amino acids are serially condensed together one by one, and the fragment condensation method in which fragments consisting of several such amino acids are synthesized in advance and serially coupled, based on the amino acid sequence information provided by the invention. Synthesis of the peptides according to the invention can be carried out by whichever of the above methods.

The condensation reactions in the above method of peptide synthesis may also be carried out by various known methods. Specifically, the azide method, mixed acid anhydride method, DCC method, active ester method, redox method, DPPA (diphenylphosphoryl azide) method, DCC + additive (1-hydroxybenzotriazole, N-hydroxysuccinimide, N-hydroxy-5-norbornene-2,3-dicarboximide) method, and Woodward's method can be mentioned by way of example. The solvent which can be utilized in these methods may also be selected judiciously from among the well-known common solvents for use in peptide condensation reactions. To mention a few examples, dimethylformamide (DMF), dimethyl sulfoxide (DMSO), hexaphosphoramide, dioxane, tetrahydrofuran (THF), and ethyl acetate, inclusive of mixtures thereof, can be used.

In conducting the above reactions for peptide synthesis, any of the carboxyl groups of amino acids or peptides that should not take part in the reactions may be protected in advance, generally by esterification in the form of a lower alkyl ester, e.g. methyl ester, ethyl ester, tert-butyl ester or the like, or an aralkyl ester, e.g. benzyl ester, p-methoxybenzyl ester, p-nitrobenzyl ester or the like. The amino acid having a functional group in the side chain, for example the hydroxyl group of Tyr, may be protected with an acetyl, benzyl, benzyloxycarbonyl, tert-butyl or other group but need not necessarily be protected in advance. Furthermore, the guanidino group of Arg, for instance, may be protected with a suitable protective group such as nitro, tosyl, 2-methoxybenzenesulfonyl, methylene-2-sulfonyl, benzyloxycarbonyl, isobornyloxycarbonyl, or adamantyloxycarbonyl. Deprotection reactions for removing such protective groups from protected amino acids, peptides, or end-product peptides of the invention can also be carried out by the conventional methods, for example the catalytic reduction method or the method using any of such reagents as liquid ammonia/sodium, hydrogen fluoride, hydrogen bromide, hydrogen chloride, trifluoroacetic acid, acetic acid, formic acid, and methanesulfonic acid.

The CD-binding peptides of the invention, thus obtained, can be purified in the conventional manner as needed, namely in accordance with the procedures in routine use in the art of peptide chemistry, such as ion exchange resin treatment, partition chromatography, gel chromatography, affinity chromatography, high performance liquid chromatography (HPLC), and countercurrent distribution.

The CD-binding peptide of the invention includes not only the various peptides mentioned hereinbefore but also oligopeptides and polypeptides containing the amino acid sequence of any of said peptides as part thereof and capable of binding specifically to Crohn's disease antibody.

As such polypeptides, the polypeptides at least comprising the amino acid sequence represented by SEQ ID NO:1, SEQ ID NO:2, SEQ ID NO:3, SEQ ID NO:4, SEQ ID NO:7, SEQ ID NO:10, SEQ ID NO:12, SEQ ID NO:16, SEQ ID NO:18, SEQ ID NO:31, and SEQ ID NO:42 can be mentioned. Also included are polypeptides partially comprising modified amino acid sequence derived from the amino acid sequence of any of the above-mentioned SEQ ID NOS. by substitution, deletion or addition of one or several amino acids and capable of binding specifically to Crohn's disease antibody.

It is generally considered that the minimum number of amino acids that is necessary for an antibody to recognize an antigen in an antigen-antibody reaction is 4. Therefore, from antigenicity points of view, the number of amino acids is not particularly restricted provided that the peptide consists of not less than 4 amino acids. Though this is not critical, the number of amino acids may generally range from 4 to 700. Moreover, when a branched multiple antigenic peptide having a plurality of amino acid sequences of said CD-binding peptides as branched chains within each molecule is to be prepared as described hereinafter, the number of amino acids constituting a branched chain is preferably within the range of, for example, 9 to 14.

More particularly, polypeptides at least comprising the amino acid sequence LIAQQM of the amino acid sequence represented by SEQ ID NO:10 can be mentioned as examples. The amino acid sequence represented by SEQ ID NO:10 is the amino acid sequence of VATE-201c peptide which is the equivalent of CD1 peptide, and as polypeptides at least comprising said amino acid sequence (LIAQQM), VATE-201 peptide consisting of the amino acid sequence represented by SEQ ID NO:11, vacuolar H⁺ transport ATPase (hereinafter referred to sometimes as V-ATPase) and its subunit E can be mentioned. The amino acid sequence of subunit E of V-ATPase is shown under SEQ ID NO:14.

V-ATPase is a H⁺ pump functioning to keep acidic the internal environment of the organelle (Golgi apparatus, lysosome, secretory granules, synaptic vesicles, yeast vacuoles, etc.) belonging to the central vacuolar system of eucaryotic cells. It is known that this V-ATPase consists of 9 subunits, namely subunits A, B, C, D and E, 115 kDa subunit, 39 kDa subunit, 20 kDa subunit, and 16 kDa subunit. The primary structure of each of these subunits is already known and the structure of V-ATPase consisting of these subunits has also been presumed (Seikagaku (Biochemistry), 65, 6, pp. 413-436, June 1993).

The CD-binding peptide of the invention includes not only a polypeptide (protein) having the full-length sequence of V-ATPase consisting of said subunits but also fragments of V-ATPase (inclusive of the respective subunits and fragments of each subunit) insofar as such fragments are capable of binding specifically to Crohn's disease antibody. Moreover, the CD-binding peptide of the invention includes complexes of human vacuolar H⁺ transport ATPase subunit E with at least one subunit selected from among other subunits, namely subunit A, subunit B, subunit C, subunit D, 115 kDa subunit, 39 kDa subunit, 20 kDa subunit, and 16 kDa subunit.

The fragment of V-ATPase referred to above includes said subunit E (33 kDa polypeptide, the number of amino acids: 226), polypeptides containing the subunit E domain (inclusive of complexes of subunit E with other subunits), 7 to 226-residue polypeptides containing the 202-208 amino acid region of the amino acid sequence of said subunit E, and 14 to 226-residue polypeptides containing the 199-212 amino acid region of the amino acid sequence of subunit E. Furthermore, said V-ATPase and its fragments (e.g. complexes of subunit E with other subunits, subunit E or portions thereof) may have been modified by substitution, deletion and addition of one or several amino acids from the respective amino acid sequences insofar as these are still capable of binding specifically to Crohn's disease antibodies. These modified proteins can be invariably defined as the equivalent of V-ATPase or its fragment (e.g. subunit E).

Furthermore, as CD-binding polypeptides, polypeptides at least comprising the amino acid sequence represented by SEQ ID NO:51 may also be mentioned by way of example. The amino acid sequence represented by SEQ ID NO:51 corresponds also to the amino acid sequence of Z300 peptide (SEQ ID NO:31) which is the equivalent of CD3 peptide. As a polypeptide at least comprising this amino acid sequence, Homo sapiens kruppel-like zinc finger protein 300 (hereinafter referred to sometimes as HZF300) can be mentioned. The amino acid sequence of the HZF300 is shown under SEQ ID NO:32. Insofar as the specific binding affinity for Crohn's disease antibody is retained, the CD-binding peptide of the invention may be such that the amino acid sequence of HZF300, represented by SEQ ID NO:32, has been modified by substitution, deletion or addition of one or several amino acids. Such modified peptide includes 7 to 604-residue polypeptides containing the 129-135 amino acid region of the amino acid sequence of HZF300 and 14 to 604-residue polypeptides containing the 126-139 amino acid region of the amino acid sequence of HZF300. These modification products can be invariably defined as the equivalent of HZF300.

Furthermore, as CD-binding polypeptides, polypeptides at least comprising the amino acid sequence of L(V)GGIYXE(D)L (X represents an arbitrary amino acid residue) can be mentioned. As such polypeptides at least comprising the above amino acid sequence, CD4 peptide and all equivalents thereof, represented by SEQ ID NOS:4 and 33 to 42, are included. In addition, as a polypeptide at least comprising the above amino acid sequence, Rice seed allergen RA14 which belongs to the gene family of a rice allergen protein (alpha-amylase/trypsin inhibitor) can also be mentioned. The amino acid sequence of Rice seed allergen RA14 is shown under SEQ ID NO:46.

Insofar as the capability of binding specifically to Crohn's disease antibodies is retained, the CD-binding peptide of the invention includes modified peptides derived from the amino acid sequence of Rice seed allergen RA14 (SEQ ID NO:46) by substitution, deletion or addition of one or several amino acids. As such modification products, 8 to 165-residue polypeptides containing the 101-108 amino acid region of the amino acid sequence of Rice seed allergen RA14 (SEQ ID NO:46) and 13 to 165-residue polypeptides containing the 99-111 amino acid region of the amino acid sequence of Rice seed allergen RA14 can be mentioned. These modification products can be invariably defined as the equivalent of Rice seed allergen RA14.

Furthermore, the CD-binding peptide of the invention includes polypeptides at least comprising modified amino acid sequences derived from the amino acid sequence of SEQ ID NO:42 by substitution, deletion or addition of one or several amino acids, provided that these are capable of binding specifically to Crohn's disease antibodies. As such polypeptides, there can be mentioned Rice allergen (SEQ ID NO:43), Rice seed allergen RA5 (SEQ ID NO:44), Rice allergen RA5B precursor (SEQ ID NO:45), Rice allergen RA14B precursor (SEQ ID NO:47), and Rice seed allergen RAG2 (SEQ ID NO:48), all of which, like Rice seed allergen RA14, belong to the gene family of alpha-amylase/trypsin inhibitors. These rice allergen proteins may also have been modified by substitution, deletion or addition of one or several amino acids provided that the modified proteins are still capable of binding specifically to Crohn's disease antibody. As such equivalents of rice allergen proteins, proteins at least comprising the amino acid sequence of L(V)GGIYREL (SEQ ID NOS:49 or 50) locating in the 95-110 amino acid region of the respective rice allergen proteins can be mentioned. More particularly, 8 to 157-residue polypeptides containing the 99-106 amino acid region of the amino acid sequence of Rice allergen, 8 to 157-residue polypeptides containing the 99-106 amino acid region of the amino acid sequence of Rice seed allergen RA5, 8 to 160-residue polypeptides containing the 102-109 amino acid region of the amino acid sequence of Rice allergen RA5B precursor, 8 to 166-residue polypeptides containing the 102-109 amino acid region of the amino acid sequence of Rice allergen RA14B precursor, and 8 to 166-residue polypeptides containing the 102-109 amino acid region of Rice seed allergen RAG2 can be mentioned. These modified proteins can be defined as the equivalents of Rice allergen, Rice seed allergen RA5, Rice allergen RA5B precursor, Rice allergen RA14B precursor, and Rice seed allergen RAG2, respectively.

Thus, V-ATPase, its subunit E (SEQ ID NO:14), and equivalents thereof belong to said equivalent of CD1 peptide; HZF300 (SEQ ID NO:32) and its equivalent belong to said equivalent of CD3 peptide; and various rice allergen proteins constituting the gene family of alpha-amylase/trypsin inhibitor (SEQ ID NOS:43 to 48) and equivalents thereof belong to said equivalent of CD4 peptide, thus being invariably subsumed in the concept of CD-binding peptide according to the present invention.

Furthermore, the CD-binding peptide of the invention may assume the form of a multiple antigen peptide (also referred to as a MAP peptide or a branched multiple antigenic peptide). This MAP peptide is characterized in that the amino acid sequences of peptides represented by SEQ ID NOS:1 to 4 (CD1 peptide to CD4 peptide) or equivalents thereof are attached to a main chain as side chains (branched chains) in a plural number in the manner of branches. The number of branched chains having the amino acid sequences of such CD-binding peptides is not particularly restricted but is preferably equal to 2 to 16, more preferably equal to 4 to 16, still more preferably equal to 8.

One preferred example of the CD-binding peptide in this MAP form according to the invention (branched multiple antigenic peptide) is a peptide having a basal molecule (skeleton) of the dendrimer structure.

The dendrimer is generally acknowledged to be a spheroidal or otherwise-configured molecule having a dendritic (tree-like) to stellate three-dimensional structure. This molecule is also characterized by its plurality of branches having functional groups (repeating units). (cf. Japanese published un-examined patent application No.S60-500295; Japanese published un-examined patent application No.S63-99233; Japanese published un-examined patent application No.H03-263431; USP 4507466; USP 4568737; Polymer Journal, 17, p. 117 (1985); Angewandte Chem. Int. Engl., 29, 138-175 (1990); Macromolecules, 25, p. 3247 (1992), etc.).

The dendrimer which can be utilized in the invention is not particularly restricted provided that it has a nuclear structure serving as an origin, an inner layer (generations) consisting of repeating units (branches) attached to said nucleus serving as the origin, and an outer surface comprising functional groups existing as attached to the respective branches. The size, shape, reactivity, etc. of said dendrimer can be controlled by judicious choice of the nucleus or origin, the number of generations, and the repeating unit to be used for each generation and these variables are not particularly restricted, either. Production of a dendrimer of appropriate size can be carried out in the conventional manner to be described hereinafter and dendrimers having different sizes can be easily obtained by increasing the number of generations to be utilized (e.g. USP 4694064).

One example of the CD-binding peptide having a dendrimer structure (branched multiple antigenic peptide) according to the invention is a polypeptide comprising a nitrogen atom as the origin or nucleus, plurality of repeating units (branches) of the -CH₂CH₂CONHCH₂CH₂-structure being attached to this nucleus, and amino acid sequences of aforementioned CD-binding peptides being attached to the outermost ends of the said respective branches. Another example of the branched multiple antigenic peptide is a polypeptide comprising an amino acid, such as Lys, Arg, Glu or Asp, as said origin or nucleus, the similar amino acid as mentioned above as the repeating unit (branch) directly attached to the said origin, and the amino acid sequence of a CD-binding peptide being attached to the terminus of each repeating unit (branch) as described above.

The above dendrimer having a nitrogen atom as the origin or nucleus can be produced in the conventional manner. Moreover, its components (dendrimer materials) are also available commercially (Polysciences, Inc., 400 Vally Road, Warrington, PA, 18976 U.S.A.). The other kind of dendrimer having an amino acid as the origin or nucleus can be produced typically in accordance with the above-mentioned technology for peptide synthesis. Moreover, it can be produced by utilizing a commercial dendrimer material such as Fmoc8-Lys4-Lys2-Lys-βAla-Alko resin (product of Watanabe Chemical Industry).

More particularly, said dendrimer material can be produced in the following manner. Thus, a protected a,?-diamino acid as protected with the same or different two amino-protective groups in advance is condensed to a resin for solid-phase peptide synthesis with or without use of a spacer, the amino-protective groups are then removed. Such condensation of the protected a,?-diamino acids and deprotection reactions are repeated.

As the resin for solid-phase peptide synthesis, the resins which are in routine use for peptide synthesis can be invariably employed. For example, a polystyrene resin, polyacrylamide resin or poly(styrene-co-ethylene glycol) resin having a chloromethyl, 4-(hydroxymethyl)phenoxy, or 4-((a-2',4'-dimethoxyphenyl)-9-fluorenylmethoxycarbonylaminomethyl)phenoxy group as the terminal group can be mentioned. The spacer may for example be one or several amino acids. The a,?-diamino acid mentioned above includes lysine, ornithine, 1,4-diaminobutyric acid, and 1,3-diaminopropionic acid, among others.

The protective group referred to above includes Boc, Fmoc, and Z groups, among others. The functional group includes amino, carboxyl and hydroxyl groups, among others. The reaction for removal of protective groups can be conducted in accordance with the above-mentioned technology for peptide synthesis. The number of branches is 2n as the condensation of the repeating unit and removal of the protective groups are carried out in n repeats. The preferred number of branches may be within the range of 2 to 16.

By coupling the amino acid sequence of a CD-binding peptide to the terminus of each branch of the dendrimer material thus obtained, the peptide in the desired MAP form (branched multiple antigen peptide) according to the invention can be acquired. This coupling reaction can be carried out by the technology for peptide synthesis described hereinbefore.

The MAP-form peptide (branched multiple antigenic peptide) of the invention can be purified by the routine procedure, such as chromatography using a suitable resin such as Sephacryl S-300 (product of Pharmacia) or other resin, as stationary phase.

Referring, further, to the branched multiple antigenic peptide of the invention, the amino acid sequences constituting the terminal structures of the respective branches need not be uniform but may reflect an arbitrary combination of the amino acid sequences of dissimilar CD-binding peptides. An example is the combination of the amino acid sequences of two or more dissimilar peptides, i.e. at least 2, preferably 3 or more dissimilar peptides, more preferably 4 or more dissimilar peptides selected from at least 2, preferably 3, more preferably 4 of the following four groups: (i) CD1 peptide and its equivalent, (ii) CD2 peptide and its equivalent, (iii) CD3 peptide and its equivalent, and (iv) CD4 peptide and its equivalent. With use of such a composite branched multiple antigenic peptide, Crohn's disease in an individual subjent can be more accurately detected.

The CD-binding peptide of the invention has the property to selectively recognize and bind to Crohn's disease-specific antibody (the antibody characteristically detected in patients with Crohn's disease). Therefore, the CD-binding peptide and the branched multiple antigenic peptide comprising the amino acid sequences thereof according to the invention can be used successfully in detecting Crohn's disease, that is to say the examination and diagnosis of Crohn's disease.

### (2) Crohn's disease examination reagents and reagent kit

The present invention provides a Crohn's disease examination reagent comprising said CD-binding peptide or said branched multiple antigenic peptide containing the amino acid sequences thereof as an active ingredient.

More particularly, the Crohn's disease examination reagent of the invention comprises, as an active ingredient, at least one CD-binding peptide selected from the group consisting of (i) said CD1 peptide and its equivalent, (ii) said CD2 peptide and its equivalent, (iii) said CD3 peptide and its equivalent, and (iv) said CD4 peptide and its equivalent, or a branched multiple antigenic peptide containing plural amino acid sequences of at least one kind of CD-binding peptide selected from (i) said CD1 peptide or equivalent, (ii) said CD2 peptide or equivalent, (iii) said CD3 peptide or equivalent, and (iv) said CD4 peptide or equivalent, the same or different, per molecule in a branching manner.

The above-mentioned equivalent of CD1 peptide includes complexes of subunit E of human vacuolar H⁺ transport ATPase (V-ATPase) with at least one unit selected from the group consisting of the other subunits of the V-ATPase, namely subunit A, subunit B, subunit C, subunit D, 115kDa subunit, 39 kDa subunit, 20 kDa subunit, and 16 kDa subunit.

The CD-binding peptide or branched multiple antigenic peptide used as the active ingredient plays the role of an antigen which binds to the Crohn's disease antibody present in a biological sample from a subject to thereby capture or label the antibody through the utilization of its specific binding affinity for Crohn's disease antibody.

As the active ingredient of a Crohn's disease examination reagent, either one species or optionally two or more species of said CD-binding peptide can be used. For improved accuracy (reliability of examination), it is preferable to use two or more species of said peptide in combination. The mode of such combination is not particularly restricted but the preferred mode of combination is the mode of using 2 or more, preferably 3 or more, more preferably 4 or more dissimilar peptides selected from among at least 2, preferably 3, more preferably 4 of the following groups (i) to (iv), viz. (i) CD1 peptide or its equivalent (e.g. CDP-1a peptide, CDP-1 peptide, CD5 peptide, CDP-5a peptide, CDP-5 peptide, VATE-201c peptide, VATE-201 peptide, CD1s peptide, CDP-1s peptide, V-ATPase, subunit E, and equivalent thereof), (ii) CD2 peptide or its equivalent (e.g. CDP-2 peptide, CD2s peptide, CDP-2s peptide, CD2s1 peptide, CDP-2s1 peptide, and equivalent thereof), (iii) CD3 peptide or its equivalent (e.g. CDP-3 peptide, CDP3 peptide, CDP3-1 peptide, CDP3-2 peptide, CDP3-3 peptide, CDP3-4 peptide, CDP3-5 peptide, CDP3-6 peptide, CDP3-8 peptide, CDP3-12 peptide, CDP3-14 peptide, Z300 peptide, human nuclear peptide (HZF300) or its equivalent, and (iv) CD4 peptide or its equivalent (e.g. CDP-4 peptide, CDP4 peptide, CDP4-1 peptide, CDP4-2 peptide, CDP4-3 peptide, CDP4-4 peptide, CDP4-10 peptide, CDP4-13 peptide, CDP4-14 peptide, TO3965 peptide, Rice allergen, Rice seed allergen RA5, Rice allergen RA5B precursor, Rice seed allergen RA14, Rice allergen RA14B precursor, Rice seed allergen RAG2, or equivalents thereof).

Referring to the case where the Crohn's disease examination reagent of the invention is a composition containing two or more kinds of CD-binding peptides as above, the formulating ratio of such CD-binding peptides is not particularly restricted. For example, such CD-binding peptides may be formulated in equal proportions or, in the case where the composition contains a CD-binding peptide with weak reactivity (binding affinity) to Crohn's disease antibody, in such a manner that the proportion of the less reactive peptide will be relatively increased. In the case where, though not restrictive, said (i) CD1 peptide, (ii) CD2 peptide, (iii) CD3 peptide, and (iv) CD4 peptide belonging to said CD-binding peptide groups (i), (ii), (iii), and (iv), respectively, are used as active ingredients, for instance, these peptides may be used in a ratio of 1:2:2:1.

In the case where the branched multiple antigenic peptide described above is used as the active ingredient of the Crohn's disease examination reagent, this multiple antigenic peptide may be such that the amino acid sequences of its branches are uniform, i.e. the amino acid sequences of one and the same CD-binding peptide, or varying, i.e. the amino acid sequences of two or more kinds of CD-binding peptides. In the latter case, the mode of combination of the amino acid sequences of CD-binding peptides to be used for said branches is not particularly restricted, either, but the preferred is the mode of using the amino acid sequences of 2 or more, preferably 3 or more, more preferably 4 or more kinds of CD-binding peptides selected from among at least 2, preferably 3, more preferably 4 of the above-mentioned groups (i) to (iv).

The Crohn's disease examination reagent of the invention, as an antigen capable of binding Crohn's disease antibody specifically, can be used for capturing or labeling of Crohn's disease antibody. Therefore, unless this object is departed from, the examination reagent of the invention may consist solely in one or more kinds of CD-binding peptides or a branched multiple antigenic peptide or may contain other additional ingredients. Moreover, for the purpose of labeling Crohn's disease antibodies, such CD-binding peptides or branched multiple antigen peptides are preferably labeled with a suitable labeling substance. The labeling substance which can be used for this purpose is not particularly restricted but the labeling substances in broad use in the art can be liberally selected and used. Among such substances are radioisotopes such as ³H and ¹⁴C; enzymes such as alkaline phosphatase, peroxidase (POX), microperoxidase, chymotrypsinogen, procarboxypeptidase, glyceraldehyde-3-phosphate dehydrogenase, amylase, phosphorylase, D-nase and P-nase; fluorescent substances such as fluorescein isothiocyanate (FITC) and tetramethylrhodamine isothiocyanate (RITC), etc.; and 1N-(2,2,6,6-tetramethyl-1-oxyl-4-piperidyl)-5N-(aspartate)-2,4-dinitrobenzene (TOPA), dye sols, metal sols, latex particles, and others. It should be understood that the CD-binding peptide or branched multiple antigen peptide according to the invention includes such labeled peptides as well.

Generally, to perform a Crohn's disease examination using a biological sample from a subject as the examination sample, it is convenient to use an examination kit containing said examination reagent comprising said CD-binding peptide or multiple antigenic peptide. Accordingly, the present invention, in another aspect, provides an examination kit which can be effectively utilized in the examination (diagnosis) of Crohn's disease.

The Crohn's disease examination kit according to the invention may be one containing said examination reagent as a capturing (trapping) agent for Crohn's disease antibody or as a labeling substance for Crohn's disease antibody, insofar as it is contained to bind Crohn's disease antibody. In the case where said examination reagent is to be utilized as a capturing agent for Crohn's disease antibodies, the reagent can be used in the form immobilized on an arbitrary support (solid phase). When it is to be utilized as a labeling substance for Crohn's disease antibodies, it is advisable to use the CD-binding peptide or multiple antigen peptide labeled with a suitable labeling substance as described above.

The other components to be used in combination with said examination reagent in the examination kit of the invention can be judiciously chosen in the routine matter according to the immunoassay technique and detection means for use in the examination of Crohn's disease. Preferably, as an additional component other than said examination reagent comprising said CD-binding peptide or multiple antigenic peptide, a secondary antibody (for example, an anti-human IgG antibody) for detecting human IgG may be included. This anti-human IgG antibody may be labeled with said labeling substance or immobilized on an arbitrary support (solid phase) in advance.

Furthermore, the examination kit may further include a substrate corresponding to the labeling substance or a detection reagent for detecting the reaction between the labeling substance and its substrate and even, for convenience in performing a determination, a suitable sample diluent, a secondary antibody diluent (e. g. an anti-human IgG antibody diluent), a standard antibody, a buffer, a washing solution, an enzyme substrate solution, and a reaction stop solution, among others. Furthermore, in the case where the examination reagent or the anti-human IgG antibody is an unlabeled one or an un-immobilized one, the examination kit may be supplemented with a labeling substance or a support (solid phase).

Thus, the Crohn's disease examination kit according to the invention is a set of reagents for the diagnosis of Crohn's disease which contains an examination reagent comprising said CD-binding peptide or said branched multiple antigenic peptide (optionally immobilized or/and labeled) as an active ingredient in combination with at least one component selected from among an anti-human IgG antibody which may optionally be immobilized or labeled, a substrate for the labeling substance, an antibody diluent, a standard antibody, a buffer solution, a washing solution, a solvent for the substrate, a reaction stop solution, a support (solid phase), and a labeling substance. From convenience, safety, and sensitivity points of view, the preferred labeling substance is an enzyme. From this point of view, the Crohn's disease examination kit according to the invention is a set of reagents for the diagnosis of Crohn's disease which contains a detection reagent comprising said CD-binding peptide or said branched multiple antigenic peptide (optionally immobilized or/and labeled) as an active ingredient in combination with at least one component selected from the group consisting of an anti-human IgG antibody which may optionally be immobilized or enzyme-labeled, a substrate for the enzyme, an antibody diluent, a standard antibody, a buffer solution, a washing solution, a solvent for the enzyme substrate, an enzymatic reaction stop solution, a support (solid phase), and an enzyme as a labeling substance. As the enzyme for use in said labeling with an enzyme, not only the above-mentioned enzymes but also such other enzymes as microperoxidase, chymotrypsinogen, procarboxypeptidase, glyceraldehyde-3-phosphate dehydrogenase, amylase, phosphorylase, D-nase, and P-nase can be mentioned by way of illustration.

### (3) Crohn's disease examination method

The present invention further provides a Crohn's disease examination method. This examination method comprises using a biological sample from a subject as the examination sample and detecting Crohn's disease in the subject. More particularly, the Crohn's disease examination method according to the invention comprises detecting Crohn's disease using the presence/absence of a specific antibody occurring specifically in a biological sample from a patient with Crohn's disease as a marker.

The examination sample includes various biological samples, such as the blood (serum, plasma), urine, sweat, saliva, seminal fluid, spinal fluid, and other samples from a subject (a human being), preferably a serum sample.

As the Crohn's disease examination method, the following three examination protocols (3-1) to (3-3) can be mentioned.

### (3-1) As will be seen from the working examples given hereinafter, an antibody which recognizes human vacuolar H⁺ transport ATPase exists specifically in patients with Crohn's disease.

Therefore, as the Crohn's disease examination method of the invention, a protocol comprising a step of detecting the antibody recognizing human vacuolar H⁺ transport ATPase (V-ATPase) in a biological sample from a subject can be mentioned. The object antibody to be detected is preferably an antibody recognizing subunit E of V-ATPase, more preferably an antibody recognizing the 199-212 amino acid region of subunit E of V-ATPase.

Detection of such antibodies can be performed by the hitherto-known immunoassay technique utilizing an antigen-antibody reaction. More particularly, the protocol comprises collecting a biological sample, preferably a serum sample, from a subject suspected to have Crohn's disease, causing the biological sample to react with an antigen capable of binding said antibody recognizing V-ATPase, and detecting the complex formed by an antigen-antibody reaction.

The antigen capable of binding to the antibody recognizing V-ATPase, mentioned just above, is not particularly restricted provided that it specifically binds to the antibody recognizing V-ATPase. Preferably, it is an antigen having the property to specifically bind to the antibody recognizing subunit E of V-ATPase and more preferably it is an antigen having the property to specifically bind the antibody recognizing the 199-212 amino acid region of subunit E of V-ATPase. As an antigen having the above property, a peptide consisting of the amino acid sequence of LIAQQM and its equivalent can be mentioned. This equivalent includes peptides consisting of modified amino acid sequences derived from the above amino sequence (LIAQQM) by deletion, substitution or addition of one or several amino acids and capable of binding specifically to an antibody recognizing subunit E of V-ATPase. Among such peptides are peptides consisting of the amino acid sequences represented by SEQ ID NO:1 and NOS:5 to 15 (CD1 peptide, CDP-1a peptide, CDP-1 peptide, CD5 peptide, CDP-5a peptide, CDP-5 peptide, VATE-201c peptide, VATE-201 peptide, CD1s peptide, CDP-1s peptide, V-ATPase, and subunit E of V-ATPase). However, this is not an exclusive list but other 6 to 226-residue peptides, preferably 6 to 14-residue peptides, which comprise the above amino acid sequence (LIAQQM), for instance, can also be used.

As the antigen substance capable of binding to an antibody recognizing V-ATPase, the complex of subunit E of V-ATPase with at least one subunit selected from subunit A, subunit B, subunit C, subunit D, 115kDa subunit, 39 kDa subunit, 20 kDa subunit, and 16 kDa subunit of the V-ATPase can also be used. In addition, as the antigen substance capable of binding to an antibody recognizing V-ATPase, a branched multiple antigenic peptide containing the peptide comprising the above amino acid sequence (LIAQQM) or its equivalent in a plurality of units, which may be the same or different, within each molecule can also be used.

### (3-2) Furthermore, as indicated in Example 6 to be presented hereinafter, an antibody recognizing a human nuclear protein (Homo sapiens kruppel-like zinc finger protein 300 (HZF300)) exists specifically in patients with Crohn's disease.

Accordingly, the Crohn's disease examination method of the invention includes an examination protocol which comprises a step of using a biological sample from a subject as the examination sample and detecting an antibody recognizing the human nuclear protein (HZF300) therein. The subject antibody to be detected is preferably an antibody recognizing the 126-138 amino acid region of HZF300.

Detection of said antibody can be performed by the hitherto-known immunoassay technique utilizing an antigen-antibody reaction. More particularly, the procedure comprises collecting a biological sample, preferably a serum sample, from a subject suspected to have Crohn's disease, causing the biological sample to react with an antigen substance capable of binding to said antibody recognizing the human nuclear protein (HZF300), and detecting the complex formed by an antigen-antibody reaction.

The above antigen substance having a binding affinity for an antibody recognizing said human nuclear protein (HZF300) is not particularly restricted provided that it is specifically bound to the antibody recognizing HZF300. Preferably the antigen has the property to specifically bind to an antibody recognizing the 126-138 amino acid region of the human nuclear protein (HZF300). As antigens having the above property, the peptide consisting of the amino acid sequence represented by SEQ ID NO:51 and its equivalent can be mentioned. The equivalent mentioned above includes any peptide or protein consisting of an amino acid sequence derived from the above sequence of SEQ ID NO:51 by addition of one or several amino acids and capable of binding specifically to an antibody recognizing the human nuclear protein (HZF300). Among such peptides are those consisting of the amino acid sequences represented by SEQ ID NO:3 and NOS:20 to 32 (CD3 peptide, CDP-3 peptide, CDP3-1 peptide, CDP3-2 peptide, CDP3-3 peptide, CDP3-4 peptide, CDP3-5 peptide, CDP3-6 peptide, CDP3-8 peptide, CDP3-12 peptide, CDP3-14 peptide, Z300 peptide, and human nuclear protein (HZF300)). However, this is not an exclusive list but other 7 to 604-residue polypeptides partially comprising the amino acid sequence of SEQ ID NO:51, for instance, can also be used.

Furthermore, as the antigen substance binding to an antibody recognizing the human nuclear protein (HZF300), a branched multiple antigenic peptide containing the peptide consisting of the amino acid sequence of SEQ ID NO:51 or its equivalent in a plurality of units, which may be the same or different, within each molecule can also be used.

### (3-3) In addition, as indicated in Example 7 to be presented hereinafter, an antibody recognizing a rice allergen protein exists specifically in patients with Crohn's disease.

Accordingly, the Crohn's disease examination method of the invention includes an examination protocol comprising a step of using a biological sample from a subject as the examination sample and detecting the antibody recognizing the rice allergen protein therein. In this connection, said rice allergen protein includes those proteins belonging to the gene family of a-amylase/trypsin inhibitors, specifically Rice allergen (158aa), Rice seed allergen RA5 (157aa), Rice allergen RA5B precursor (160aa), Rice seed allergen RA14 (165aa), Rice allergen RA14B precursor (166aa), and Rice seed allergen RAG2 (166aa), among others. The subject antibody to be detected is preferably an antibody which recognizes at least the amino acid sequence of L(or V)GGIYREL in the 95-110 amino acid region of such various rice allergen proteins.

Detection of such antibodies can be performed by the hitherto-known immunoassay technique utilizing an antigen-antibody reaction. More particularly, the procedure comprises collecting a biological sample, preferably a serum sample, from a subject suspected to have Crohn's disease, causing the biological sample to react with an antigen substance capable of binding to said antibody recognizing the said rice allergen protein, and detecting the complex formed by an antigen-antibody reaction.

The antigen substance which binds to an antibody recognizing said rice allergen protein is not particularly restricted provided that it is specifically bound to an antibody recognizing a protein belonging to the gene family of said rice allergen protein (a-amylase/trypsin inhibitor). The preferred antigen substance is one having the property to specifically bind antibodies recognizing at least the amino acid sequence of L(or V)GGIYREL (SEQ ID NOS:49 or 50) in the 95-110 amino acid region of various rice allergen proteins.

As specific examples of the antigen substance having the above property, the peptide consisting of the amino acid sequence of L(V)GGIYXD(E)L (X represents an arbitrary amino acid residue) or its equivalent can be mentioned. The equivalent mentioned just above includes peptides consisting of amino acid sequences derived from the above-mentioned sequence of L(V)GGIYXD(E)L (X represents an arbitrary amino acid residue) by addition of one or several amino acids and capable of binding specifically to an antibody recognizing the rice allergen protein. Among such peptides are peptides consisting of the amino acid sequences represented by SEQ ID NOS:4 and 33 to 48 (CD4 peptide, CDP-4 peptide, CDP4-1 peptide, CDP4-2 peptide, CDP4-3 peptide, CDP4-4 peptide, CDP4-10 peptide, CDP4-13 peptide, CDP4-14 peptide, TO3965 peptide, Rice allergen, Rice seed allergen RA5, Rice allergen RA5B precursor, Rice seed allergen RA14, Rice allergen RA14B precursor, and Rice seed allergen RAG2). However, this is not an exclusive list but other 8 to 166-residue polypeptides, preferably 8 to 14-residue polypeptides, which comprise the amino acid sequence of L(V)GGIYXD(E)L (X represents an arbitrary amino acid residue) can also be used.

Furthermore, as the antigen substance binding to an antibody which recognizes the rice allergen protein, a branched multiple antigenic peptide containing the peptide consisting of the above amino acid sequence of L(V)GGIYXD(E)L (X represents an arbitrary amino acid residue) or its equivalent in a plurality of units, which may be of the same or different, within each molecule can also be used.

The method of detecting the antigen-antibody complex resulting from the reaction between such an antigen substance and the antibody specifically existing in a Crohn's disease patient is not particularly restricted but the routine techniques can be liberally adopted. More particularly, an immunoassay technique utilizing any of said various CD-binding peptides and branched multiple antigenic peptides comprising the said peptides as the antigen substance can be mentioned as one preferred example. Taking the solid-phase sandwich assay technique using a human serum as the examination sample as an example, the object antibody can be assayed by the following procedure, for instance.

First, the above-mentioned peptide for use as the antigen substance is immobilized (the resulting artifact is hereinafter referred to briefly as "solid-phase peptide") and a biological sample (e.g. a serum sample) as the examination sample is added. Thereupon, an antigen-antibody reaction takes place between the solid-phase peptide and the Crohn's disease patient-specific antibody in the examination sample, so that the object antibody in the examination sample is bound to the solid-phase peptide. Then, the presence/absence of the bound antibody and its amount (antibody titer) are detected with a human antibody (IgG) detection reagent. In this manner, the object antibody in the examination sample, that is a biological sample (e.g. serum) from a subject, can be detected and quantitated.

In connection with the above technique, the object antibody specific to a Crohn's disease patient in the examination sample can also be detected and, at the same time, its amount (antibody titer) determined by immobilizing said human antibody (IgG) detection reagent in advance, then adding the examination sample (biological sample) thereto so as to capture the object antibody occurring in the biological sample, and adding said antigen substance to let it be bound to the object antibody. Furthermore, by adding a specific antibody against the antigen substance bound to the object antibody as above to cause the specific antibody to be bound to the antigen substance, the antibody specific to the Crohn's disease patient can be detected and its titer determined by utilizing this antibody as a marker. Selection and modification of various means used in such assay techniques are well known to those skilled in the art and any of such techniques can be invariably employed in the practice of the invention (cf. Handbook of Clinical Examination Methods, Kanehara Shuppan, 1995, for instance).

The human antibody (IgG) detection reagent for detecting Crohn's disease antibodies is not particularly restricted but various reagents in routine use can be utilized. For example, anti-human IgG antibodies specifically binding to human IgG can be used with advantage. Although such antibodies can be purchased from commercial sources, these can be prepared by the conventional method.

In the case where the object antibody is to be detected using such a human antibody (IgG) detection reagent as a marker, preferably this human antibody (IgG) detection reagent has been labeled. The labeling substance for such labeling includes radioisotopes such as ³H, ¹⁴C, etc.; enzymes such as alkaline phosphatase, peroxidase (POX), etc.; fluorescent substances such as fluorescein isothiocyanate (FITC), tetramethylrhodamine isothiocyanate (RITC), etc.; and 1N-(2,2,6,6-tetramethyl-1-oxyl-4-piperidyl)-5N-(aspartate)-2,4-dinitrobenzene (TOPA), dye sols, metal sols, latex particles, and so forth. The immunoassay techniques using detection reagents labeled with such labeling substances are known as radioimmunoassay, enzyme immunoassay, fluoroimmunoassay, spin immunoassay, flow-through immunoassay, and immunochromatoassay, respectively.

In the present invention, from convenience, safety, sensitivity and other points of view, the enzyme immunoassay technique using an enzyme as the labeling substance is preferably used. The enzyme which can be used for this labeling with an enzyme includes, in addition to the enzymes mentioned above, such enzymes as microperoxidase, chymotrypsinogen, procarboxypeptidase, glyceraldehyde-3-phosphate dehydrogenase, amylase, phosphorylase, D-nase, and P-nase, among others. The labeling with such a labeling substance can be carried out in the per se known manner (Iwasaki, T. et al.: Monoclonal Antibody, Kodansha Scientific, 1984; Ishikawa, E. et al.: Enzyme Immunoassay, 2nd edition, Igaku Shoin, 1982; etc.).

In the case where the object antibody is to be detected and assayed by using an antigen substance (said CD-binding peptide or said branched multiple antigenic peptide containing amino acid sequences thereof) as a marker, it is preferable to use the labeled peptide as the antigen substance. The labeling of such an antigen substance can also be carried out in the routine manner using a suitable labeling substance just as for the labeling of anti-human IgG antibodies.

When the solid-phase method is adopted in the above assay protocol, the antigen substance or the anti-human IgG antibody may be immobilized on a support (solid phase) in advance and used for capturing the object antibody. The support is not particularly restricted provided that it is an insoluble inert matrix and, as such matrix, the materials in common use can be liberally selected and used. Thus, sticks, beads, microplates, test tubes, etc. of various raw materials such as glass, cellulose powder, Sephadex, Sepharose, polystyrene, filter paper, carboxymethylcellulose, ion exchange resins, dextran, plastic film, plastic tubing, nylon, glass beads, silk, polyamine-methyl vinyl ether-maleic acid copolymer, amino acid copolymers, ethylene-maleic acid copolymer, etc. can be liberally selected and used.

The method of immobilizing the antigen or the anti-human IgG antibody on a solid phase is not particularly restricted, either, but whichever of physical coupling and chemical coupling can be utilized. More particularly, there can be mentioned covalent binding methods, such as diazo method, peptide method (acid amide derivative method, carboxyl-chloride resin method, carbodiimide resin method, maleic anhydride derivative method, isocyanate derivative method, cyanogen bromide-activated polysaccharide method, cellulose carbonate derivative method, method using a condensing reagent, etc.), alkylation method, support binding method using a crosslinking agent (glutaraldehyde or hexamethylene isocyanate, for instance, is used as the crosslinking agent), support binding method using Ugi reaction and other methods using chemical reactions; the ionic binding method using an ion exchange resin or the like as the matrix, and the physical adsorption method using a porous glass matrix such as glass beads.

As the solvent for use in the above assay system, any of the common solvents that will not adversely affect the reaction can be utilized. More particularly, buffer solutions from pH about 5 to pH about 9, such as citrate buffer, phosphate buffer, Tris-HCl buffer, acetate buffer, etc., can be mentioned by way of illustration.

The immune reaction conditions (conditions of said antigen-antibody reaction) are not particularly restricted but the conditions in common use for assay systems of this kind can be adopted. Generally speaking, the reaction can be conducted at a temperature below 45°C, preferably at a temperature of about 4-40°C, for about 1-40 hours.

Determination of the antigen-antibody complex formed by said antigen-antibody reaction can be carried out in the conventional manner depending on the kind of lableing substance used.

In the case where an enzyme is used as a labeling substance, for instance, the object can be accomplished by measuring the activity of the enzyme. Measurement of enzyme activity can be carried out in the known manner according to the kind of enzyme used. For example, when a peroxidase is used as the enzyme, ABTS [2,2'-azinobis(3-ethylbenzothiazolinesulfonic acid)] is used as the substrate or when alkaline phosphatase is used, p-nitrophenyl phosphate is used as the substrate, and after incubation, the degree of degradation of the substrate is measured with a spectrometer, for instance [e.g. Ishikawa, E. et al.: Enzyme Immunoassay 2nd, Igaku Shorin, 1982]. When a radioisotope or a fluorescent substance is used as the label, too, measurements can be carried out by the respective known techniques.

### [Examples]

The following examples illustrate the present invention in further detail without defining the scope of the invention.

### Example 1 Selection of Crohn's disease antibody-binding peptide and its identification

### (1) Preparation of a phage display library

A phage display library (1.0 x 10E8 clones) was constructed by the method reported by Franco et al. (Franco Felici. et al., J. Mol. Biol., 222, 301-310 (1991)) with some modification. More particularly, this phage display library is a filamentous phage with a DNA containing a sequence of NNK (N is any of A, C, G and T, and K is G or T) in 9 repeats having been inserted by genetic engineering and, in addition, a DNA coding for a peptide consisting of 9 random amino acid residues having been inserted into the N-terminal region of the main capsid (coat) protein pVIII gene so that the peptide having an amino acid sequence of 9 random residues may be expressed on the surface of the phage capsid.

### (2) Selection of Crohn's disease antibody-binding peptides (CD-binding peptides)

### (i) Immobilization of the serum antibody

As the antibody, serum antibody was used. As to the serum, 20 serum samples from patients with Crohn's disease, and, as control sera, 20 serum samples from ulcerative colitis patients and 20 serum samples from healthy volunteers were used.

The antibody (IgG) in the serum was immobilized on magnetic beads in the following manner. To magnetic beads (Dynabeads M-450, Tosyl-activated) was added an anti-human IgG(Fc)-specific antibody (Biodesign), prepared in a concentration of 200 pg/ml with 0.1 M borate buffer, and the reaction was carried out at 4°C overnight. After the reaction, the magnetic beads were washed with (D)-PBS (Dulbecco's phosphate buffer) containing 0.1% of bovine serum albumin (BSA) and subjected to blocking with 0.1% BSA-containing 0.2 M Tris(2-amino-2-hydroxymethyl-1,3-propanediol) (Tris-HCl) and, then, 0.1% BSA-containing (D)-PBS to prepare anti-human IgG(Fc)-specific antibody-carrying magnetic beads.

Then, to the anti-human IgG(Fc)-specific antibody-carrying magnetic beads, serum samples for 3 cases randomly selected from 20 Crohn's disease patients (CDG1 and CDG2) and serum samples for 5 cases randomly selected from 20 healthy volunteers were respectively added and the reaction was carried out overnight to prepare magnetic beads on the surface of which the corresponding serum IgG (healthy subject serum IgG or Crohn's disease patient serum IgG) had been immobilized via the anti-human IgG(Fc)-specific antibody.

### (ii) Selection of CD-binding peptide display phages (biopanning)

To the magnetic beads supporting the healthy volunteer serum IgG, an about 1 x 10¹¹ phage library (a library such that a sequence of random 9 amino acids is displayed in the pVIII region of M13 phage) was added and the reaction was carried out at 4°C overnight. Then, the unbound phage was added to the magnetic beads supporting the Crohn's disease patient serum IgG and the reaction was carried out at 4°C overnight. After the reaction, the beads were washed with 0.1% BSA-containing (D)-PBS and, thereafter, the phage bound to the beads was eluted with an elution buffer (1 mg/ml BSA-containing 0.1 M HCl adjusted to pH 2.2 with glycine). The eluted phage was neutralized with 1M Tris and the neutralized phage was caused to infect *Escherichia coli* JM109. The infected cells were inoculated onto LB agar medium containing 150 µg/ml ampicillin and 1% glucose and cultured at 37°C overnight. After culture, the grown *E. coli* cells on the medium were thoroughly collected by scraping, infected with a helper phage (M13KO7), and, after addition of IPTG (isopropyl-β-D(-)-thiogalactopyranoside) and kanamycin, shake culture was carried out at 37°C overnight. The culture broth was centrifuged to remove insolubles, a solution of sodium chloride containing polyethylene glycol was added, and after several stirrings, the broth was recentrifuged and the pellet was recovered and dissolved in 0.02% sodium azide-containing (D)-PBS to give a concentrated phage solution.

Using the phage solution thus obtained, the above biopanning was repeated twice. The phage solution obtained by the third biopanning was used to infect *Escherichia coli* JM109 and the infected cells were inoculated onto LB agar medium containing 150 µg/ml ampicillin and 1% glucose and cultured at 37°C overnight. The haploid colonies of *E. coli* were recovered by scraping and shake-cultured in LB liquid medium containing 150 µg/ml ampicillin at 37°C for 3 hours. After infection with a helper phage (M13KO7) and addition of IPTG and kanamycin, shake culture was carried out at 37°C overnight.

In this manner, a monoclonal CD-binding peptide display phage was obtained.

### (iii) Phage ELISA

Using the monoclonal CD-binding peptide display phage obtained as above, phage ELISA was carried out with the same healthy volunteer serum pool and Crohn's disease patient serum pool as used in the above biopanning (cf. (i)).

In the ELISA, an anti-phage antibody (Pharmacia) was immobilized on a 96-well microtiter plate in the first place. Specifically, this immobilization was carried out by adding an anti-phage antibody (Pharmacia) solution prepared in a 1 µg/ml concentration with (D)-PBS, to the plate, 100 µl per well. The plate was left sitting at 4°C overnight and then washed, and after addition of 300 µl of blocking solution ((D)-PBS containing 1% BSA and 5% sorbitol), further left sitting at 4°C overnight.

The primary reaction was carried out as follows. Thus, 10 µl of the phage solution in (ii) was added to 90 µl of phage ELISA buffer ((D)-PBS containing 1% BSA, 0.05% Tween 20 and 10% normal goat serum), the mixture was added to each well coated with said anti-phage antibody, and the reaction was conducted at 37°C for 1 hour. After completion of the primary reaction, the plate was washed 4 times and the secondary reaction was carried out. The secondary reaction was performed by adding the mixture containing 1 µl of serum (healthy volunteer serum or Crohn's disease patient serum) and 100 µl of phage ELISA buffer, to each well, and allowing the reaction to proceed at 37°C for 1 hour. After completion of the secondary reaction, the plate was washed 4 times and the tertiary reaction was carried out. The tertiary reaction was conducted by adding HRP (horseradish peroxidase)-labeled anti-human IgG (Fc)-specific antibody, diluted 40,000-fold in phage ELISA buffer (20 ng/ml) in advance, to the plate, 100 µl per well, and allowing the reaction to proceed at 37°C for one hour. After completion of the tertiary reaction, the plate was washed and a color development reaction was carried out. The color development was conducted by adding TMB (3,3',5,5'-tetramethylbenzidine) solution to the plate, allowing the reaction to proceed at room temperature for 10 minutes, and stopping the reaction with a stopper (1N sulfuric acid). The plate after reaction stopping was measured for absorbance (OD 450 nm) with a plate reader to evaluate the reactivity with the serum antibody.

Based on the results, phage clones not reacting to the healthy volunteer serum antibody but reacting exclusively to the Crohn's disease patient serum antibody were selected. Then, for each of the selected phage clones, ELISA was carried out with 20 Crohn's disease patient serum samples, 20 ulcerative colitis patient serum samples and 20 healthy volunteer serum samples, and based on the reactivities, 5 clones showing high specificity to Crohn's disease patient serum (CD-1, CD-2, CD-3, CD-4, CD-5) were selected. For the 5 clones thus selected, the reactivity (ELISA) with the respective serum samples (Crohn's disease patient serum, ulcerative colitis patient serum, and healthy volunteer serum) was evaluated. The results are shown in Fig. 1.

### (3) Determination of the amino acid sequences of CD-binding peptides

The amino acid sequences of the above 5 clones selected by phage ELISA (CD-1, CD-2, CD-3, CD-4, CD-5) were determined. First, DNA was extracted from the selected phage clones. More particularly, *Escherichia coli* JM109 was used to infect each clone and inoculated on ampicillin-containing LB agar medium and cultured overnight. The colonies formed on the medium were collected by scraping and shake-cultured in 2 ml of ampicillin-containing LB liquid medium overnight and the plasmid DNA was extracted using Qiaprep DNA extraction kit (Qiagen).

Determination of the base sequence of phage DNA was carried out by the dideoxy method (Proc. Natl. Acad. Sci., USA, 74, 5463-5467 (1977)) using Amersham's Cycle Sequencing Kit (Amersham Pharmacia Biotech, Code; 2438) in accordance with the kit manual. The DNA sequencing was carried out using Pharmacia's DNA sequencer (ALF DNA Sequencer).

The amino acid sequences of respective clones (CD-1, CD-2, CD-3, CD-4, CD-5) as deduced from the base sequences found are shown in the one-letter expression format in Table 5.

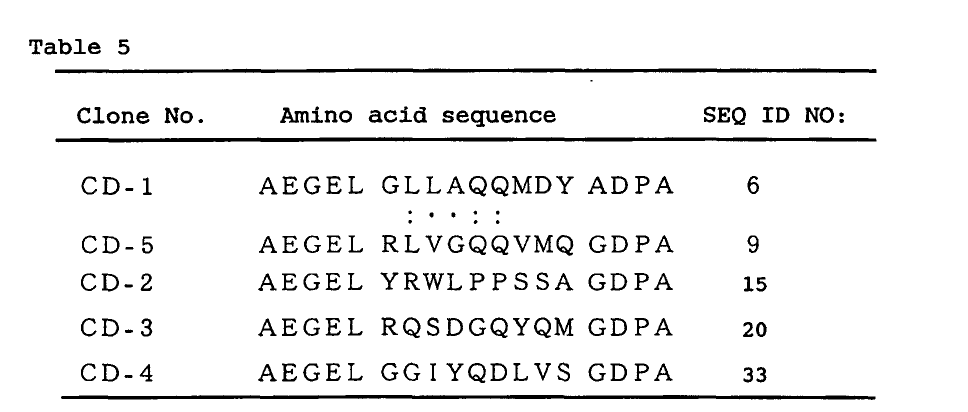

In the above table, AEGEL and either ADPA or GDPA at the N-terminal region and C-terminal region, respectively, of each peptide are derived from the corresponding terminal amino acid sequences of the random peptide of the phage vector.

Using the said a 18-residue peptide containing amino acid sequence derived from the phage vector, for each of the CD-1, CD-2, CD-3, and CD-4 clones excepting CD-5 clone, was synthesized in the form of a branched multiple antigenic peptide (MAP peptide) and used in the following experiment. Thus, using the commercial Fmoc8-Lys4-Lys-2-βAla-Alko (product of Watanabe Chemical Industry), peptides having the under-mentioned amino acid sequences were synthesized by means of ATC-357 peptide synthesizer (manufactured by Advanced ChemTech).
CDP-1: AEGELGLLAQQMDYADPA (SEQ ID NO:6)
CDP-2: AEGELYRWLPPSSAGDPA (SEQ ID NO:15)
CDP-3: AEGELRQSDGQYQMGDPA (SEQ ID NO:20)
CDP-4: AEGELGGIYQDLVSGDPA (SEQ ID NO:33)

As synthesized by this method of synthesis, each branched multiple antigenic peptide (MAP peptide) has 8 amino acid sequences of the CD-binding peptide per molecule in a dendritic pattern (Fig. 2).

### Example 2 Reactivity of CD-binding peptides to serum samples

### (1) ELISA using each branched multiple antigenic peptide (MAP peptide) as the antigen

Using each MAP peptide (the MAP peptides of CDP-1, CDP-2, CDP-3, and CDP-4; see Fig. 2) obtained in Example 1 as the antigen peptide, the reactivity to each serum sample (Crohn's disease patient serum, ulcerative colitis patient serum, and healthy volunteer serum) was evaluated by ELISA.

In the first place, each MAP peptide was immobilized on a 96-well microtiter plate. Thus, this immobilization was carried out in accordance with the protocol which comprises dissolving each MAP peptide in bicarbonate buffer (50 mM, pH 9.6) at a concentration of 1 µg/ml to prepare a MAP solution, adding the solution to the antigen plate, 100 µl per well, then leaving the plate sitting at 4°C overnight, washing it, adding 300 µl of casein solution ((D)-PBS containing 0.1% casein and 1% Triton X-100), and leaving the plate sitting again at 4°C overnight.
(i) Using the MAP plates carrying the respective MAP peptides immobilized, the reactivity of each MAP peptide to 20 Crohn's disease patient serum samples, 20 ulcerative colitis patient serum samples and 48 healthy volunteer serum samples was verified by ELISA.
   More particularly, a primary reaction was carried out by adding mixture containing 1 µl of the serum antibody and 100 µl of casein solution to each well of the MAP plate, and allowing the reaction to proceed at 37°C for one hour. After completion of the primary reaction, the plate was washed 4 times and a 20,000-fold dilution of HRP-labeled anti-human IgG(Fc)-specific antibody in casein solution was added into the wells and incubated at 37°C for one hour to carry out a secondary reaction. After completion of the secondary reaction, the plate was washed 4 times and a color development reaction was carried out. For detection, 100 µl of TMB solution was added to the plate and reacted at room temperature for 10 minutes, at the end of which time the reaction was stopped by adding 100 µl of TMB stop solution (1N sulfuric acid). The absorbance at OD 450 nm was measured with a plate reader to investigate the reactivity to each MAP plate for each serum. The results are shown in Fig. 3. It will be apparent from Fig. 3 that any of the 4 kinds of MAP peptides (the MAP peptides of CDP-1, CDP-2, CDP-3, and CDP-4) did not appreciably react with ulcerative colitis patient sera or healthy volunteer sera but reacted with 20-40 percent of Crohn's disease patient sera. It can also be seen in Fig. 3 that the reactivity of each MAP peptide to Crohn's disease patient serum varied among different kinds of MAP peptides without consistency. These findings suggested that these 4 peptides are different types of peptides with Crohn's disease patient-specific reactivity.
(ii) Then, using a large number of serum samples (96 Crohn's disease patient serum samples, 20 ulcerative colitis patient serum samples, and 48 healthy volunteer serum samples), the reactivity of the above MAP peptides (the MAP peptides of CDP-1, CDP-2, CDP-3, and CDP-4) was investigated by the same method as in (i) to evaluate the specificity of the respective peptides to Crohn's disease patient sera. The results are presented in Table 6.

**Table 6**

| MAP peptide | Positive rate | positive rate | |
|---|---|---|---|
| | Crohn's disease patients | Ulcerative colitis patients | Healthy volunteers |
| CDP-1 | 31.3% (30/96) | 0% (0/20) | 4.2% (2/48) |
| CDP-2 | 27.1% (26/96) | 5% (1/20) | 2.1% (1/48) |
| CDP-3 | 51.0% (49/96) | 0% (0/20) | 0 % (0/48) |
| CDP-4 | 31.3% (30/96) | 5% (1/20) | 4.2% (2/48) |

It will be apparent from Table 6 that, when the mean OD value + 5SD for 48 healthy volunteer serum samples was taken as the cut-off value, the positive rates for 96 Crohn's disease patient serum samples were found to be as follows: CDP-1 peptide 31.3%, CDP-2 peptide 27.1%, CDP-3 peptide 51.0%, and CDP-4 peptide 31.3%. The positive rates for healthy volunteer sera and ulcerative colitis patient sera were not over 5% with all the peptides. These results suggested that the respective peptides (CDP-1 peptide, CDP-2 peptide, CDP-3 peptide and CDP-4 peptide) are specifically bound to the antibody specifically present in patients with Crohn's disease (Crohn's disease antibody) and that by taking advantage of this reaction, Crohn's disease can be accurately diagnosed.

### (2) ELISA using a mixed MAP peptide as the antigen

The MAP peptides prepared in Example 1 (the MAP peptides of CDP-1, CDP-2, CDP-3, and CDP-4) were mixed to prepare a mixed MAP peptide and by using this mixed MAP peptide as the antigen, the reactivity to various serum samples (Crohn's disease patient sera, ulcerative colitis patient sera, duodenal ulcer patient sera, gastric ulcer patient sera and healthy volunteer sera) was investigated by ELISA.

The antigen plate used for detection was provided by immobilizing a mixture, which is prepared by mixed 1.5 µg/ml each of CDP-1 MAP peptide and CDP-4 MAP peptide and 3 µg/ml each of CDP-2 MAP peptide and CDP-3 MAP peptide in equal parts, on a 96-well microtiter plate in the same manner as in the preparation of the MAP plate described in (1).
(i) Using this mixed antigen plate, the reactivity of the mixed MAP peptide to various sera was investigated by ELISA using 550 Crohn's disease patient serum samples, 20 ulcerative colitis patient serum samples, 120 healthy volunteer serum samples, 25 duodenal ulcer patient serum samples, and 15 gastric ulcer patient serum samples. As to the ELISA, except that a 10,000-fold dilution in casein solution ((D)-PBS containing 0.1% casein and 1% Triton X-100) was used as the HRP-labeled anti-human IgG(Fc)-specific antibody for the secondary reaction, the procedure described in the above paragraph (1)(i) was repeated. The results are presented in Fig. 4 and Table 7.

**Table 7**

| | | Cut-off value | |
|---|---|---|---|
| | | Healthy volunteer mean + 5SD | Healthy volunteer mean + 3SD |
| Positive rate | Crohn's disease patients | 61.3%(337/550) | 67.1%(369/550) |
| positive rate | Ulcerative colitis patients | 5.0% (1/20) | 5.0% (1/20) |
| | Healthy volunteers | 0.8% (1/120) | 1.7% (2/120) |
| | Duodenal ulcer patients | 0% (0/25) | 0% (0/25) |
| | Gastric ulcer patients | 0% (0/15) | 0% (0/15) |

It will be apparent from Fig. 4 and Table 7 that when the mean unit value + 3SD for 120 healthy volunteer serum samples was taken as the cut-off value, the positive rate for Crohn's disease patient sera was 67.1% (369/550) and the positive rates were 5% (1/20) for ulcerative colitis patient sera, 1.7% (2/120) for healthy volunteer sera, and 0% (0/25 or 0/15) for duodenal ulcer patient sera or gastric ulcer patient sera.

Comparison of the above results with the results obtained in (1) suggested that compared with the exclusive use of each Crohn's disease-binding peptide (CDP-1 peptide, CDP-2 peptide, CDP-3 peptide, or CDP-4 peptide), the combined use of these peptides leads to an improved specificity to the Crohn's disease-specific antibody, thus enabling a more accurate diagnosis of Crohn's disease. (3) Comparison of a mixed MAP peptide with baker's yeast as the antigen in ELISA

Just as in (2), using a mixture of the MAP peptides prepared in Example 1 (the MAP peptides of CDP-1, CDP-2, CDP-3 and CDP-4) as the antigen, the reactivity to various serum samples (Crohn's disease patient sera, ulcerative colitis patient sera and healthy volunteer sera) was investigated by ELISA. At the same time, using baker's yeast *(Saccharomyces cerevisiae*)(Gut. 1998, 42, pp. 788-791, Gastroenterology, 1999, 116, pp. 1001-1003, Am. J. Gastroenterol., 2001, 96, pp. 730-734), whose relationship to Crohn's disease had been pointed out, too, the reactivity to the above serum samples was investigated to evaluate the relative usefulness of the two antigens in the diagnosis of Crohn's disease. The mixed MAP plate used as an antigen plate was prepared as follows. Thus, the MAP peptide of CDP-1 and the MAP peptide of CDP-4 were prepared each in a concentration of 1.5 µg/ml and the MAP peptide of CDP-2 and the MAP peptide of CDP-3 were prepared each in a concentration of 3 µg/ml and these are mixed in equal parts and immobilized on a 96-well microtiter plate in the same manner as the preparation of the MAP plate in (1). The reactivity of baker's yeast was determined with the commercial *Anti-Saccharomyces cerevisiae* antibody detection kits (ASCA IgG detection kit and ASCA IgA detection kit; antigen used: glucomannan of *S. cerevisiae* cell membrane, product of Medizyme).
(i) Using the above mixed MAP plate, the reactivity of 96 Crohn's disease patient serum samples, 20 ulcerative colitis patient samples and 48 healthy volunteer serum samples to mixed MAP was confirmed by ELISA.
(ii) Moreover, the reactivity to baker's yeast was confirmed in accordance with the manuals included in the assay kits. The results are presented in Fig. 5. In the ELISA using the mixed MAP peptide as an antigen, the mean unit value + 3SD for 48 healthy volunteer serum samples was used as the cut-off value as shown in Fig. 5A, while in the ELISA with the assay kits using ASCA IgG or ASCA IgA as antigens, the binding index = 1.0 was used as the cut-off value in accordance with the kits' manuals as shown in Fig.5B or 5C. Then, the positive rates were calculated. The calculated positive rates are shown in Table 8.

**Table 8**

| Antigen | Positive rate | positive rate | |
|---|---|---|---|
| | Crohn's disease patients | Ulcerative colitis patients | Healthy volunteers |
| Mixed MAP | 66.7%(64/96) | 5.0%(1/20) | 2.1%(1/48) |
| ASCA IgG | 30.2%(29/96) | 10.0%(2/20) | 10.4%(5/48) |
| ASCA IgA | 13.5%(13/96) | 5.0%(1/20) | 0%(0/48) |

These results suggested that as compared with the use of baker's yeast which is acknowledged to be an antigen recognizing Crohn's disease antibody, the use of various MAP peptides in the form of mixed MAP peptide enables the more specific recognition of Crohn's disease antibody and more accurate diagnosis of Crohn's disease.

### Example 3 Homology analysis of CD-binding peptides

For the MAP peptides obtained (the MAP peptides of CDP-1 to CDP-4), their homology in amino acid sequence to the proteins reportedly related to Crohn's disease [CDX (measles related antigen)(Gut. 2000 Feb;46(2):163-9), porcine pancreatic alpha-amylase (Annual Report of the Research Committee of Inflammatory Bowel Disease, Japan: The Ministry of Health and Welfare of Japan, 1999:98-100), *M. paratuberculosis* HSP65 (horseradish peroxidase 65)(Clin. Diagn. Lab. Immunol. 1995, Nov;2(6):657-64), human HSP60 [Digestion, 1997;58(5):469-75], *M. paratuberculosis* p36 (Curr. Microbiol., 1999 Aug;39(2):115-9)] was analyzed by means of DNASIS software (Hitachi Ltd.). The results are shown in Fig. 6. It will be apparent from the results that a weak homology but no high homology was noted with each protein.

Then, for the amino acid sequences of the MAP peptides, a database search was performed for proteins having amino acid sequence homologous to the Crohn's disease antibody-binding peptides using FASTA Program (Genome Net Site used). The search was made in such a manner that only high-homology proteins with Z-scores not less than 130 should be sampled. The results are shown in Fig. 7. It will be apparent from Fig. 7 that as far as CD1 peptide, CD3 peptide, and CD4 peptide are concerned, their homology was found not only to proteins from yeast or mycobacterium, whose association with Crohn's disease has heretofore been reported, but also to proteins from a wide range of living species such as bacteria, animals, plants, etc., inclusive of pathogenic microorganism and foods (e.g. Zea myze) of which the relation to Crohn's disease has not heretofore been recognized.

### Example 4

In view of the reactivity to various sera as determined by phage ELISA (Fig. 1) and the similarity in amino acid sequence (Table 2) as found in Example 1, the peptides having the amino acid sequences of CD-1 and CD-5 clones (CDP-1 peptide (SEQ ID NO:6) and CDP-5 peptide (SEQ ID NO:9)) were considered to recognize the same antibody. Moreover, the CDP-1 peptide and CDP-5 peptide have homology in amino acid sequence to the peptide (VATE-201 peptide, SEQ ID NO:11) located in the 199-212 amino acid region of subunit E of human vacuolar H⁺ transport ATPase (V-ATPase) (Fig. 8).

Therefore, the following peptides (CDP-1a peptide, CDP-5a peptide, VATE-201 peptide) were respectively synthesized in the MAP form (multiple antigenic peptides) by the same procedure as described in Example 1(3) (Fig. 9), and in accordance with the protocol of Example 2(1), the reactivity of each MAP peptide to various serum samples (Crohn's disease patient sera, ulcerative colitis patient sera and healthy volunteer sera) was investigated by ELISA.
CDP-1a: AEGELGLLAQQMDYADP (SEQ ID NO:5)
CDP-5a: AEGELRLVGQQVMQGDP (SEQ ID NO:8)
VATE-201: RLDLIAQQMMPEVR (SEQ ID NO:11)

As test sera, 20 Crohn's disease patient serum samples were used and, as control sera, 20 ulcerative colitis serum samples and 20 healthy volunteer serum samples were used. The results are presented in Fig. 10. It will be seen in Fig. 10 that CDP-1a peptide, CDP-5a peptide and VATE-201 peptide showed ELISA reactivities mutually alike.

### Example 5 Test for inhibition of antigen-antibody reactions using VATE-201 peptide

To confirm the reactivity of VATE-201 obtained in Example 4, a test for inhibition of the antigen-antibody reaction between CDP-1a peptide and Crohn's disease antibody was carried out using VATE-201 peptide. As serum antibody samples (Crohn's disease antibody samples), the Crohn's disease patient serum No. 8, No. 9 and No. 14 (cf. Fig. 11) which showed strong reactivity to the respective MAP peptides (CDP-1a peptide, CDP-5a peptide, VATE-201 peptide) were used.

More particularly, the MAP plate prepared by immobilizing CDP-1a MAP peptide was used as the antigen plate (see Example 2(1)). On the other hand, 1 µL of each serum sample was added to 100 µL of a casein solution ((D)-PBS containing 0.1% casein and 1% Triton X-100) containing the MAP peptide of VATE-201 (reaction inhibitor) at a concentration of 100 µg/ml and the reaction was carried out at 37°C for one hour. The whole amount of the reaction mixture thus obtained was added to the above MAP plate and ELISA was performed in the same manner as in Example 2(1). As a comparative experiment, using the MAP peptide of CDP-1a in lieu of the MAP peptide of VATE-201 as said reaction inhibitor, ELISA was carried out in otherwise the same manner. The results are shown in Fig. 11.

As indicated at B in Fig. 11, addition of the MAP peptide of VATE-201 to the reaction systems invariably inhibited the reactivity of Crohn's disease serum samples (No. 8, No. 9, No. 14) with the MAP plate immobilizing CDP-1a MAP peptide. Similar results were also obtained in a similar experiment using the MAP peptide of CDP-5a in lieu of the above MAP peptide of CDP-1a, as said reaction inhibitor (Data not shown).

Thus, the results of Examples 4 and 5 indicated that all the VATE-201 peptide, CDP-1a peptide and CDP-5a peptide recognize the antibody specifically present in the sera of patients with Crohn's disease. Moreover, from the similarity to VATE-201 peptide in amino acid sequence and from the commonality to VATE-201 peptide in the reactivity to serum antibodies, it is suspected that the CD1 peptide and its equivalent, inclusive of CDP-1a peptide and CDP-5a peptide, simulate or mimic subunit E of human vacuolar H⁺ transport ATPase (V-ATPase).

Thus, the results of the above examples indicate that antibodies to human vacuolar H⁺ transport ATPase subunit E are specifically present in patients with Crohn's disease. This finding, in turn, suggests that by using the antibody recognizing human vacuolar H⁺ transport ATPase, particularly its subunit E, as a marker, Crohn's disease can be diagnosed in each individual.

### Example 6 Search for proteins simulated or mimicked by CD3 peptide and its equivalent and evaluation thereof

Based on the results of Example 5, CD1 peptide and its equivalent were found to simulate or mimic subunit E of human vacuolar H⁺ transport ATPase (V-ATPase). In this example, a search was made for proteins simulated or mimicked by CD3 peptide and its equivalent as well and the reactivity of the proteins to Crohn's disease antibodies was evaluated.

### (1) Preparation of MAP peptides

As modification products of CDP3 peptide, 14 kinds of peptides were prepared by substituting alanine for one amino acid residue each in the amino acid sequence of CDP3 peptide (SEQ ID NO:21)(Table 9). As to CDP3-1, of which the first amino acid is alanine, serine was substituted for the alanine. Then, using these 15 kinds of peptides, MAP peptides having 8 peptides per molecule were prepared in accordance with the procedure described in Example 1(3) (one amino acid-substituted MAP peptides).

### (2) Determination of CDP3 epitope sequence

Referring to the amino acid sequence of CDP peptide, in order to determine the sequence necessary for the specific reactivity to Crohn's disease antibody, the following reaction inhibition test was performed using each of the one amino acid-substituted MAP peptides prepared as above as the reaction inhibitor. As the serum antibody samples for this reaction, the Crohn's patient serum No. 2, No. 7 and No. 8 which showed strong reactivity with CDP-3 peptide in Example 2 were used (Fig. 3).

More particularly, using the MAP plate prepared by immobilizing the MAP peptide of CDP3 (see Example 2(1)) as the antigen plate, the test was performed by 3 reaction steps. In the primary reaction, the Crohn's disease patient serum was diluted 100-fold with a sample diluent containing the one amino acid-substituted MAP peptide in a concentration of 100 µg/mL (0.1 M Tris-buffer containing 0.5 M sodium chloride, 1.5% casein, 2% normal goat serum and 0.2% Tween 20) and reacted at 25°C for one hour. In the secondary reaction, 100 µL/well of the primary reaction mixture was added to the plate and reacted at 25°C for one hour, after which the plate was washed 3 times. In the tertiary reaction, 100 µL/well of HRP-labeled anti-human IgG (Fc)-specific antibody diluted 5,000-fold in an enzyme-labeled antibody diluent (Tris buffer containing 0.14 M sodium chloride, 0.5% BAS, 5% normal goat serum and 0.05% Tween 20) was added to the plate, and reacted at 25°C for 1 hour, followed by 3 rounds of washing. Detection was performed as in Example 2(1) to measure the reaction-inhibiting activity of the one amino acid-substituted MAP peptide. The results are shown in Table 9

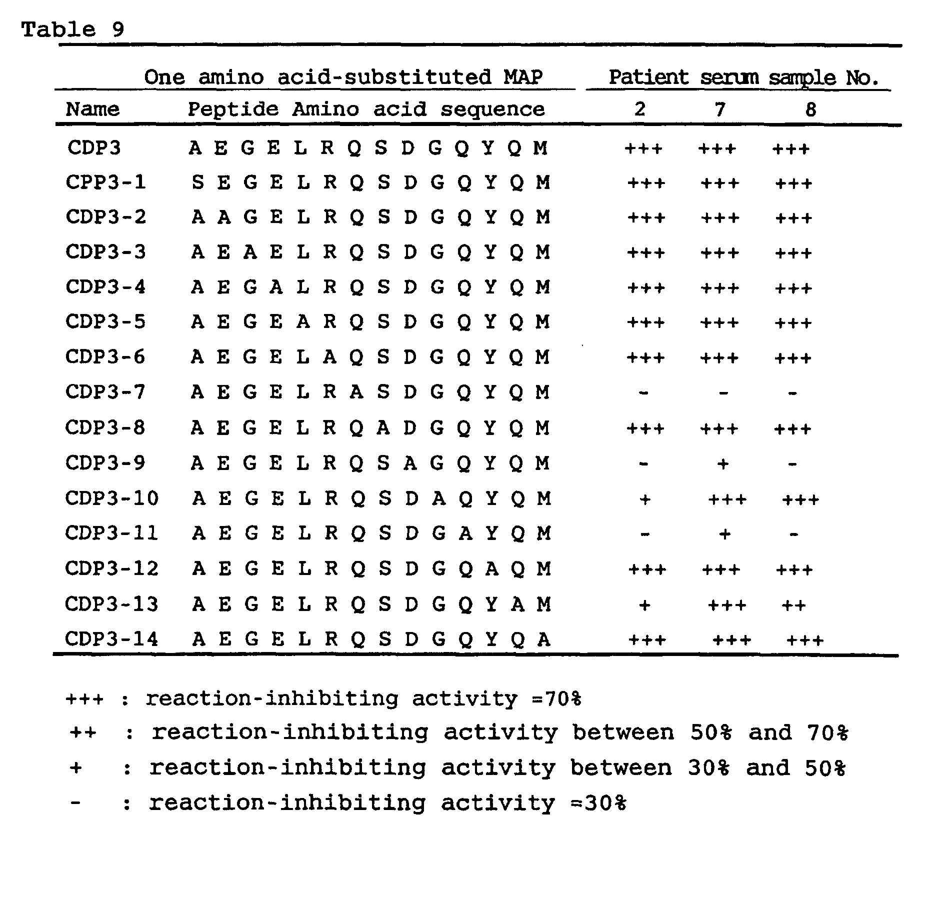

Thus, when Crohn's disease patient serum No. 2 was used as the test serum and the MAP peptides of CDP3-7, CDP3-9, CDP3-10, CDP3-11 and CDP3-13 were respectively used as the reaction inhibitor, the inhibitory activity against the reaction between Crohn's disease patient serum and CDP3 peptide was found to be not greater than 50%. When Crohn's disease patient serum No. 7 or No. 8 was used as the test serum and the MAP peptides of CDP3-7, CDP3-9 and CDP3-11 were respectively used as said reaction inhibitor, the inhibitory activity against the reaction between Crohn's disease patient serum and CDP3 peptide was found to be not greater than 50%. Therefore, in the amino acid sequence of CDP3, the sequence necessary for the reaction with Crohn's disease antibody (recognition of antibody) was considered to be QXDGQXQ (X may be the same or different and represents an arbitrary amino acid residue) (SEQ ID NO:51).

### (3) Search for proteins simulated or mimicked by CDP3 peptide

Based on the above finding, a protein database homology analysis was carried out using the amino acid sequence (QXDGQXQ (X is the same or different and represents an arbitrary amino acid residue)), which is considered to be important to recognition of Crohn's disease antibody. As a result, it was confirmed that the above amino acid sequence has homology to the 129-135 amino acid region of human nuclear protein (Homo sapiens kruppel-like zinc finger protein 300 (HZF300))(Fig. 12).

Therefore, in order to confirm that human nuclear protein (HZF300) is a protein simulated or mimicked by CDP3 peptide, the peptide (Z300 peptide) having the amino acid sequence of the 126-138 amino acid region of HZF300 was prepared in the form of a MAP peptide and the MAP antigen ELISA was carried out using 20 Crohn's disease patient serum samples, 20 ulcerative colitis patient serum samples, and 20 healthy volunteer serum samples.

### (4) MAP antigen-ELISA

First, each human serum was diluted 101-fold with the sample diluent (described hereinbefore) and 100 µL/well of the solution was added to the MAP plate prepared by immobilizing the said MAP peptide. Then, the procedure of the secondary reaction and subsequent operation described in Example 6(2) was followed to investigate the reactivity of the MAP peptide to various kinds of serum antibodies. The results are shown in Fig. 13. It was found that Crohn's disease patient serum No. 2, No. 7 and No. 8 showing strong reactivity to CDP3 peptide showed similar reactivity to Z300 peptide. On the other hand, Z300 peptide did not show definite reactions with ulcerative colitis patient sera and healthy volunteer sera.

### (5) Test for inhibition of antigen-antibody reactions

To verify the reactivity of Z300 peptide to Crohn's disease antibodies, a test for inhibition of the antigen-antibody reaction between CDP3 peptide and Crohn's disease antibody was performed using Z300 peptide. As serum antibody samples, Crohn's disease patient serum No. 2, No. 7, and No. 8 showing strong reactivity to CDP3 peptide were used.

More particularly, the MAP plate prepared by immobilizing the MAP peptide of CDP3 on a plate (Example 2(1)) was used. To 100 µL of the sample diluent containing 100 µg/ml of Z300 MAP peptide (reaction inhibitor) was added 1 µL of the serum sample and the reaction was carried out at 25°C for one hour. The whole amount of this reaction mixture was added to the plate and ELISA was performed in the same manner as in Example 6(2). As a comparative experiment, ELISA was similarly performed using the MAP peptide of CDP3, in lieu of the MAP peptide of Z300, as said reaction inhibitor. The results are shown in Fig. 14.

As can be seen from Fig. 14 B, addition of the MAP peptide of Z300 to the reaction system inhibited the reactivity of all Crohn's disease patient sera (No. 2, No. 7, No.8) to the CDP3 MAP plate.

The above results indicated that both Z300 peptide and CDP3 peptide recognize the antibodies specifically present in the sera of patients with Crohn's disease. Moreover, from the similarity to Z300 peptide in amino acid sequence and from the commonality to Z300 peptide in the reactivity to serum antibody, it was considered that CD3 peptide and its equivalent according to the invention simulate or mimic human nuclear protein (Homo sapiens kruppel-like zinc finger protein 300).

Thus, the results of the above example indicate that antibodies to human nuclear protein (Homo sapiens kruppel-like zinc finger protein 300) are present specifically in patients with Crohn's disease. This finding, in turn, implies that by using an antibody recognizing human nuclear protein (Zinc finger protein 300) as a marker, Crohn's disease can be diagnosed.

### Example 7 Search for proteins simulated or mimicked by CD4 peptide and its equivalent, and evaluation thereof

Then, a search was made for proteins simulated or mimicked by CD4 peptide and its equivalent and the reactivity thereof to Crohn's disease antibodies was evaluated.

### (1) Preparation of MAP peptides

As modification products of CDP4 peptide, 14 kinds of peptides were prepared by substituting alanine for one amino acid residue each in the amino acid sequence of CDP4 peptide (SEQ ID NO:33)(Table 10). As to CDP4-1, the first amino acid of which is alanine, serine was substituted for the alanine. Then, using these 15 kinds of peptides, MAP peptides having 8 peptides per molecule were prepared in accordance with the procedure described in Example 1(3) (one amino acid-substituted MAP peptides).

### (2) Determination of CDP4 epitope sequence

Referring to the amino acid sequence of CDP4 peptide, in order to determine the sequence necessary for the specific reactivity to Crohn's disease antibody, the reaction inhibition test was performed as in Example 6(2) by using each of the one amino acid-substituted MAP peptides prepared as above as the reaction inhibitor. As the serum antibody samples for this reaction, the Crohn's patient serum No. 3, No. 6, No. 15, No. 17 and No. 20 which showed strong reactivity to CDP-4 peptide in Example 2 were used (Fig. 3). The results are shown in Table 10.

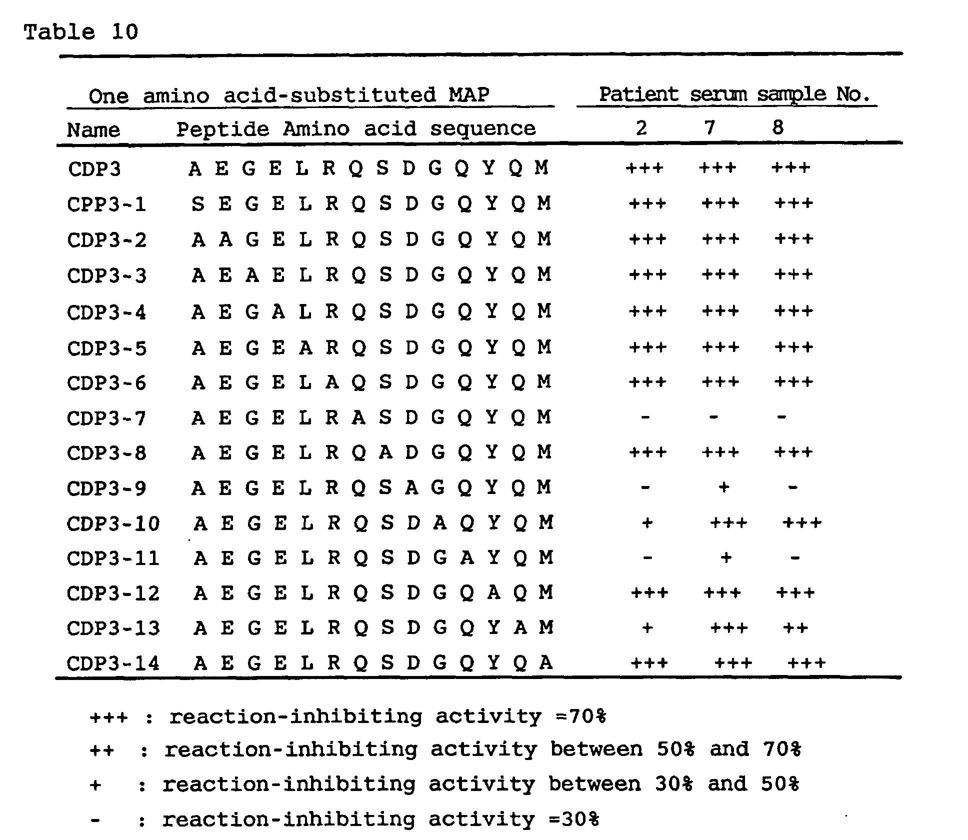

When the MAP peptides of CDP4-7, CDP4-8, CDP4-9 and CDP4-12 were respectively used as the reaction inhibitor, the inhibitory activity against the reaction between each of Crohn's disease patient serum samples No. 3, No. 6, No. 15, No. 17 and No. 20 and CDP4 peptide was not more than 30%, respectively. When the MAP peptide of CDP4-6 was used as the reaction inhibitor, the inhibitory activity against the reaction between each of Crohn's disease patient serum samples No. 6, No. 15, No. 17 and No. 20 and CDP4 peptide was not more than 30%, respectively. Furthermore, when the MAP peptides of CDP4-5 and CDP4-11 were respectively used as the reaction inhibitor, the inhibitory activity of MAP peptide of CDP4-5 or CDP4-11 against the reaction between Crohn's disease patient serum No. 6 or No.15 and CDP4 peptide, respectively, was not more than 30%.

The above results led to the conclusion that, in the amino acid sequence of CDP4 peptide, the sequence necessary for the reaction with Crohn's disease antibody (recognition of the antibody) was LGGIYXDL (X represents an arbitrary amino acid residue)(SEQ ID NO:49).

### (3) Search for proteins simulated or mimicked by CDP4 peptide

Based on the above finding, a protein database homology analysis was performed using the amino acid sequence (LGGIYXDL (X represents an arbitrary amino acid residue)) which was considered to be important to the recognition of Crohn's disease antibodies. As a result, a homology to rice allergen proteins was confirmed (Fig. 15). It can be seen in Fig. 15 that rice allergen proteins are a-amylase/trypsin inhibitors constituting a gene family. These a-amylase/trypsin inhibitors having high mutual homology in amino acid sequence were invariably found to have the amino acid sequence L(or V)GGIYXD(or E)L (SEQ ID NOS:49 or 50) which was considered to be important to the reaction with Crohn's disease antibody (recognition of the antibody). It is to be understood that L and V are alike in that both are aliphatic amino acids and D and E are alike in that both are acidic amino acids.

At this junction, in order to endorse the assumption that rice allergen proteins are proteins simulated or mimicked by CDP4 peptide, a peptide (TO3965 peptide) having an amino acid sequence corresponding to the 99-111 amino acid region of Rice seed allergen RA14 (Fig. 16) was prepared in the form of a MAP peptide and the MAP antigen-ELISA was performed using 20 Crohn's disease patient serum samples, 20 ulcerative colitis patient serum samples, and 20 healthy volunteer serum samples.

### (4) MAP antigen-ELISA

Except that a MAP antigen plate prepared by immobilizing the MAP peptide of TO3965 on the plate was used as the antigen plate, the MAP antigen-ELISA was carried out in otherwise the same manner as in Example 6(4). The results are shown in Fig. 17. Thus, Crohn's disease patient serum Nos.: 3, 6, 15, 17 and 20 showing strong reactivity to CDP4 peptide showed similar reactivity to TO3965 peptide as well. On the other hand, TO3965 peptide, like CDP4 peptide, did not show definite reactions with ulcerative colitis patient sera and healthy volunteer sera.

### (5) Test for inhibition of antigen-antibody reactions

To confirm the reactivity of TO3965 peptide to Crohn's disease antibody, a test for inhibition of the antigen-antibody reaction between CDP4 peptide and Crohn's disease antibody was performed using TO3965 peptide as a reaction inhibitor as in Example 6(5). As serum antibody samples, Crohn's disease patient serum Nos.: 3, 6, 15, 17 and 20 showing strong reactivity to CDP4 peptide were used. The results are shown in Fig. 18.

It will be apparent from Fig. 18 that addition of the MAP peptide of TO3965 to the reaction system resulted in inhibition everything of the reactivity of Crohn's disease patient serum samples (Nos.: 3, 6, 15, 17 and 20) to the CDP4 MAP plate.

The above results indicated that both TO3965 peptide and CDP4 peptide recognize the antibodies which are specifically present in the sera of patients with Crohn's disease. Moreover, the similarity to TO3965 peptide in amino acid sequence and the commonality to TO3965 peptide in the reactivity to serum antibody suggested that CD4 peptide and its equivalent according to the invention simulate or mimic rice allergen proteins (a-amylase/trypsin inhibitor gene family).

Thus, the results of the above example indicate that antibodies to rice allergen proteins (a-amylase/trypsin inhibitors) are specifically present in patients with Crohn's disease. Therefore, Crohn's disease in individuals can be diagnosed by using an antibody recognizing the gene family of rice allergen protein (a-amylase/trypsin inhibitor) as a marker.

### INDUSTRIAL APPLICABILITY

In accordance with the present invention there can be provided peptides specifically binding to antibodies present specifically in patients with Crohn's disease. As such, the peptides of the invention are useful as examination reagents for Crohn's disease, and by using these peptides and examination reagents comprising them, Crohn's disease can be accurately diagnosed.

Furthermore, the present invention provides a novel finding that antibodies recognizing human vacuolar H⁺ transport ATPase, particularly subunit E of human vacuolar H⁺ transport ATPase, antibodies recognizing the human nuclear protein (Homo sapiens kruppel-like zinc finger protein 300), and antibodies recognizing the gene family of rice allergen proteins (a-amylase/trypsin inhibitors) are specifically present in the bodies of patients with Crohn's disease. And based on this finding, the invention provides an examination method for Crohn's disease which comprises using at least one kind of antibody among said antibodies as a marker and detecting the presence/absence of the antibody in a biological sample from a subject. In accordance with the method, Crohn's disease can be expediently and accurately diagnosed by using a biological sample (such as serum) as the examination sample.

## Claims

1. A Crohn's disease antibody-binding peptide (a) or (b):
(a) a peptide consisting of an amino acid sequence selected from among the amino acid sequences represented by SEQ ID NOS:1 to 4
(b) a peptide consisting of a modified amino acid sequence derived from the above-mentioned amino acid sequence (a) by substitution, deletion or addition of one or several amino acids and capable of binding to Crohn's disease antibody.

2. The Crohn's disease antibody-binding peptide according to Claim 1, wherein the peptide (b) is a peptide partially comprising an amino acid sequence represented by any of SEQ ID NOS: 1 to 4 and SEQ ID NO:7.

3. The Crohn's disease antibody-binding peptide according to Claim 1, wherein the peptide consisting of a modified amino acid sequence derived from the amino acid sequence of SEQ ID NO:1 by substitution, deletion, or addition of one or several amino acids and capable of binding to Crohn's disease antibody is one consisting of an amino acid sequence represented by any of SEQ ID NOS:5 to 14.

4. The Crohn's disease antibody-binding peptide according to Claim 1, wherein the peptide consisting of a modified amino acid sequence derived from the amino acid sequence of SEQ ID NO:1 by substitution, deletion, or addition of one or several amino acids and capable of binding to Crohn's disease antibody is a 6 to 226-residue peptide at least comprising LIAQQM of the amino acid sequence represented by SEQ ID NO:10.

5. The Crohn's disease antibody-binding peptide according to Claim 1, wherein the peptide consisting of a modified amino acid sequence derived from the amino acid sequence of SEQ ID NO:2 by substitution, deletion, or addition of one or several amino acids and capable of binding to Crohn's disease antibody is one consisting of an amino acid sequence represented by any of SEQ ID NOS:15 to 19.

6. The Crohn's disease antibody-binding peptide according to Claim 1, wherein the peptide consisting of a modified amino acid sequence derived from the amino acid sequence of SEQ ID NO:3 by substitution, deletion, or addition of one or several amino acids and capable of binding to Crohn's disease antibody is one consisting of an amino acid sequence represented by any of SEQ ID NOS:20 to 32.

7. The Crohn's disease antibody-binding peptide according to Claim 1, wherein the peptide consisting of a modified amino acid sequence derived from the amino acid sequence of SEQ ID NO:3 by substitution, deletion, or addition of one or several amino acids and capable of binding to Crohn's disease antibody is a 7 to 604-residue peptide at least comprising the amino acid sequence represented by SEQ ID NO:51.

8. The Crohn's disease antibody-binding peptide according to Claim 1, wherein the peptide consisting of a modified amino acid sequence derived from the amino acid sequence of SEQ ID NO:4 by substitution, deletion, or addition of one or several amino acids and capable of binding to Crohn's disease antibody is one consisting of an amino acid sequence represented by any of SEQ ID NOS:33 to 48.

9. The Crohn's disease antibody-binding peptide according to Claim 1, wherein the peptide consisting of a modified amino acid sequence derived from the amino acid sequence of SEQ ID NO:4 by substitution, deletion, or addition of one or several amino acids and capable of binding to Crohn's disease antibody is an 8 to 165-residue peptide at least comprising the amino acid sequence of L(V)GGIYXD(E)L (X represents an arbitrary amino acid residue).

10. A branched multiple antigenic peptide containing the amino acid sequence of the following:
(a) a peptide consisting of an amino acid sequence selected from among the amino acid sequences represented by SEQ ID NOS:1 to 4 or
(b) a peptide consisting of a modified amino acid sequence derived from the above-mentioned amino acid sequence (a) by substitution, deletion or addition of one or several amino acids and capable of binding to Crohn's disease antibody
in a plurality of units, which may be the same or different, as branched chain sequences within each molecule.

11. The branched multiple antigenic peptide according to Claim 10, which comprises as the branched chain sequences the amino acid sequences of two or more dissimilar Crohn's disease antibody-binding peptides selected from at least two of the following groups: (i) a peptide consisting of the amino acid sequence represented by SEQ ID NO:1 and its equivalent, (ii) a peptide consisting of the amino acid sequence represented by SEQ ID NO:2 and its equivalent, (iii) a peptide consisting of the amino acid sequence represented by SEQ ID NO:3 and its equivalent, and (iv) a peptide consisting of the amino acid sequence represented by SEQ ID NO:4 and its equivalent.

12. An examination reagent for Crohn's disease which comprises as an active ingredient at least one member selected from the Crohn's disease antibody-binding peptide claimed in Claim 1; the branched multiple antigenic peptide claimed in Claim 10; and the complex of subunit E of human vacuolar H⁺-transport ATPase with at least one of the other subunits selected from subunit A, subunit B, subunit C, subunit D, 115 kDa subunit, 39 kDa subunit, 20 kDa subunit, and 16 kDa subunit.

13. The examination reagent for Crohn's disease according to Claim 12, wherein said Crohn's disease antibody-binding peptide comprises two or more dissimilar Crohn's disease antibody-binding peptides selected from at least two of the following groups: (i) a peptide consisting of the amino acid sequence represented by SEQ ID NO:1 and its equivalent, (ii) a peptide consisting of the amino acid sequence represented by SEQ ID NO:2 and its equivalent, (iii) a peptide consisting of the amino acid sequence represented by SEQ ID NO:3 and its equivalent, and (iv) a peptide consisting of the amino acid sequence represented by SEQ ID NO:4 and its equivalent, and said branched multiple antigenic peptide is the peptide claimed in Claim 11.

14. An examination kit for Crohn's disease which comprises the examination reagent claimed in Claim 12 as the antigenic substance for binding Crohn's disease antibody.

15. The examination kit according to Claim 14, comprising an anti-human IgG antibody and the examination reagent claimed in Claim 12, optionally together with at least one member selected from a sample diluent, a labeling substance, a support (solid phase), a diluent for anti-human IgG antibody, an enzyme substrate, and a reaction stop solution.

16. An examination method for Crohn's disease which comprises a step of detecting the presence of an antibody recognizing human vacuolar H⁺-transport ATPase in a biological sample from a subject.

17. The examination method for Crohn's disease according to Claim 16, wherein said antibody is an antibody which recognizes subunit E of human vacuolar H⁺-transport ATPase.

18. An examination method for Crohn's disease according to Claim 16, wherein said antibody is an antibody which recognizes the complex of subunit E of human vacuolar H⁺-transport ATPase with at least one of the other subunits selected from subunit A, subunit B, subunit C, subunit D, 115 kDa subunit, 39 kDa subunit, 20 kDa subunit, and 16 kDa subunit.

19. The examination method for Crohn's disease according to Claim 16, wherein said antibody is an antibody which recognizes the 199-212 amino acid region of subunit E of human vacuolar H⁺-transport ATPase.

20. The examination method for Crohn's disease according to Claim 16, comprising a step of using as an antigen, any of a peptide consisting of the amino acid sequence of LIAQQM or its equivalent, and a branched multiple antigenic peptide containing the amino acid sequence of said peptide or equivalent in a plurality of units, which may be the same or different, as branched chain sequences within each molecule,
and detecting a complex formed by the antigen-antibody reaction between the said antigen and the antibody recognizing human vacuolar H⁺-transport ATPase.

21. The examination method for Crohn's disease according to Claim 20, wherein the peptide consisting of the amino acid sequence of LIAQQM or its equivalent is a 6 to 227-residue peptide comprising the amino acid sequence of LIAQQM.

22. The examination method for Crohn's disease according to Claim 20, wherein said equivalent of a peptide consisting of the amino acid sequence of LIAQQM is a peptide consisting of an amino acid sequence represented by any of SEQ ID NO:1 and NOS:5 to 14.

23. The examination method for Crohn's disease according to Claim 16, comprising a step of using as an antigen, a complex of subunit E of human vacuolar H⁺-transport ATPase with at least one of the other subunits selected from subunit A, subunit B, subunit C, subunit D, 115 kDa subunit, 39 kDa subunit, 20 kDa subunit, and 16 kDa subunit,
and detecting a complex formed by the antigen-antibody reaction between the said antigen and the antibody recognizing human vacuolar H⁺-transport ATPase.

24. An examination method for Crohn's disease which comprises a step of detecting the presence of an antibody recognizing a human nuclear protein (Homo sapiens kruppel-like zinc finger protein 300) in a biological sample from a subject.

25. The examination method for Crohn's disease according to Claim 24, wherein said antibody is an antibody which recognizes the 126-138 amino acid region of the human nuclear protein (Homo sapiens kruppel-like zinc finger protein 300).

26. The examination method for Crohn's disease according to Claim 24, comprising a step of using as an antigen, any of the peptide consisting of an amino acid represented by SEQ ID NO:51 or its equivalent, and a branched multiple antigenic peptide containing the amino acid sequence of said peptide or equivalent in a plurality of units, which may be the same or different, as branched chain sequences within each molecule,
and detecting a complex formed by the antigen-antibody reaction between the said antigen and the antibody recognizing the human nuclear protein (Homo sapiens kruppel-like zinc finger protein 300).

27. The examination method for Crohn's disease according to Claim 26, wherein the peptide consisting of an amino acid sequence represented by SEQ ID NO:51 or its equivalent is a 7 to 604-residue peptide comprising the amino acid sequence represented by SEQ ID NO:51.

28. The examination method for Crohn's disease according to Claim 26, wherein said equivalent of the peptide consisting of an amino acid sequence represented by SEQ ID NO:51 is a peptide consisting of an amino acid sequence represented by any of SEQ ID NOS:3 and 21 to 32.

29. An examination method for Crohn's disease which comprises a step of detecting the presence of an antibody recognizing a rice allergen protein in a biological sample from a subject.

30. The examination method for Crohn's disease according to Claim 29, wherein said rice allergen protein belongs to the gene family of alpha-amylase/trypsin inhibitors.

31. The examination method for Crohn's disease according to Claim 29, wherein said rice allergen protein is at least one member selected from Rice allergen, Rice seed allergen RA5, Rice allergen RA5B precursor, Rice seed allergen RA14, Rice allergen RA14B precursor, and Rice seed allergen RAG2.

32. The examination method for Crohn's disease according to Claim 29, wherein said antibody recognizing the rice allergen protein is an antibody which recognizes the 99-111 amino acid region of Rice seed allergen RA14.

33. The examination method for Crohn's disease according to Claim 29, wherein said antibody recognizing the rice allergen protein is an antibody recognizing an amino acid region comprising the amino acid sequence L(V)GGIYREL of the gene family of alpha-amylase/trypsin inhibitors.

34. The examination method for Crohn's disease according to Claim 29, comprising a step of using as an antigen, any of a peptide consisting of the amino acid sequence of L(V)GGIYXD(E)L (X represents an arbitrary amino acid residue which may be the same or different) or its equivalent, and a branched multiple antigenic peptide having the amino acid sequence of said peptide or equivalent in the plurality of units, which may be the same or different, as branched chain sequences within each molecule,
and detecting a complex formed by the antigen-antibody reaction between the said antigen and the antibody recognizing the rice allergen protein.

35. The examination method for Crohn's disease according to Claim 34, wherein the peptide consisting of the amino acid sequence L(V)GGIYXD(E)L (X represents an arbitrary amino acid residue which may be the same or different) or its equivalent is an 8 to 166-residue peptide comprising the amino acid sequence: L(V)GGIYXD(E)L (X represents an arbitrary amino acid residue which may be the same or different).

36. The examination method for Crohn's disease according to Claim 34, wherein said equivalent of the peptide consisting of the amino acid sequence of L(V)GGIYXD(E)L (X represents an arbitrary amino acid residue which may be the same or different) is a peptide consisting of an amino acid sequence represented by any of SEQ ID NOS:4 and 33 to 48.

37. Use of the peptide claimed in any of Claims 1 to 11 as the antigen to be reacted with Crohn's disease antibody in an examination for Crohn's disease.

38. Use of the peptide claimed in any of Claims 1 to 11 for the manufacture of examination reagents for Crohn's disease.
